(19) Europäisches Patentamt / European Patent Office / Office européen des brevets

(11) **EP 1 791 966 B1**

(12) **EUROPEAN PATENT SPECIFICATION**

(45) Date of publication and mention of the grant of the patent:
**04.06.2014 Bulletin 2014/23**

(51) Int Cl.:
*C12N 9/28* (2006.01)    *B08B 7/00* (2006.01)
*B08B 17/02* (2006.01)    *C11D 3/386* (2006.01)

(21) Application number: **05795624.5**

(22) Date of filing: **07.09.2005**

(86) International application number:
**PCT/US2005/031813**

(87) International publication number:
**WO 2006/031554 (23.03.2006 Gazette 2006/12)**

(54) **METHODS FOR PREVENTING, REMOVING, REDUCING, OR DISRUPTING BIOFILM**

VERFAHREN ZUR VORBEUGUNG, ENTFERNUNG, VERRINGERUNG ODER UNTERBRECHUNG VON BIOFILM

PROCEDES PERMETTANT DE DETRUIRE, DE REDUIRE, D'ELIMINER OU D'EMPECHER LA FORMATION D'UN FILM BIOLOGIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.09.2004 US 608535 P**

(43) Date of publication of application:
**06.06.2007 Bulletin 2007/23**

(60) Divisional application:
**10180231.2 / 2 258 836**
**10180241.1 / 2 258 837**

(73) Proprietors:
• **Novozymes North America, Inc.**
  **Franklinton, NC 27525 (US)**
• **Novozymes A/S**
  **2880 Bagsvaerd (DK)**

(72) Inventors:
• **DEINHAMMER, Randy**
  **Wake Forest, North Carolina 27587 (US)**
• **ANDERSEN, Carsten**
  **DK-3500 Vaerlose (DK)**

(74) Representative: **Rasmussen, Preben et al**
**Novozymes A/S**
**Patents**
**Krogshoejvej 36**
**2880 Bagsvaerd (DK)**

(56) References cited:
**WO-A-00/22103          WO-A-01/98214**
**WO-A-98/26807          WO-A-99/20239**
**WO-A2-00/60060        WO-A2-01/66712**
**US-A- 5 071 765         US-B1- 6 342 386**

• **MACHIUS ET AL.: 'Kinetic stabilization of Bacillus licheniformis a-amylase through introduction of hydrophobic residues at the surface' JBC vol. 278, no. 13, 2003, pages 11546 - 11553, XP008117717**
• **YUUKI ET AL.: 'Complete nucleotide sequence of a gene coding for heat- and pH-stable a-amylase of Bacillus licheniformis: Comparison of the amino acid sequences of three bacterial liquefying a-amylases deduced from DNA sequences' J. BIOCHEM. vol. 98, 1985, pages 1147 - 1156, XP002017641**
• **KANDRA ET AL.: 'Action pattern and subsite mapping of Bacillus licheniformis a-amylase (BLA) with modified maltooligosaccharide substrates' FEBS LETTERS vol. 518, 2002, pages 79 - 82, XP004353643**

SEQUENCE LISTING

<110> Deinhammer, Randy Andersen, Carsten

<120> Methods for preventing,removing, reducing or disrupting biofilms

<130> 10648.204-WO

<160> 6

<170> PatentIn version 3.3

<210> 1
<211> 1455
<212> DNA
<213> Bacillus sp.

<220>
<221> CDS
<222> (1)..(1455)
<223> AA560

<220> mat_peptide
<221> mat_peptide
<222> (1)..()

<400> 1

```
cac cat aat ggt acg aac ggc aca atg atg cag tac ttt gaa tgg tat        48
His His Asn Gly Thr Asn Gly Thr Met Met Gln Tyr Phe Glu Trp Tyr
1                   5                   10                  15

cta cca aat gac gga aac cat tgg aat aga tta agg tct gat gca agt        96
Leu Pro Asn Asp Gly Asn His Trp Asn Arg Leu Arg Ser Asp Ala Ser
                20                  25                  30

aac cta aaa gat aaa ggg atc tca gcg gtt tgg att cct cct gca tgg       144
Asn Leu Lys Asp Lys Gly Ile Ser Ala Val Trp Ile Pro Pro Ala Trp
            35                  40                  45

aag ggt gcc tct caa aat gat gtg ggg tat ggt gct tat gat ctg tat       192
Lys Gly Ala Ser Gln Asn Asp Val Gly Tyr Gly Ala Tyr Asp Leu Tyr
        50                  55                  60

gat tta gga gaa ttc aat caa aaa gga acc att cgt aca aaa tat gga       240
Asp Leu Gly Glu Phe Asn Gln Lys Gly Thr Ile Arg Thr Lys Tyr Gly
65                  70                  75                  80

acg cgc aat cag tta caa gct gca gtt aac gcc ttg aaa agt aat gga       288
Thr Arg Asn Gln Leu Gln Ala Ala Val Asn Ala Leu Lys Ser Asn Gly
                85                  90                  95

att caa gtg tat ggc gat gtt gta atg aat cat aaa ggg gga gca gac       336
Ile Gln Val Tyr Gly Asp Val Val Met Asn His Lys Gly Gly Ala Asp
                100                 105                 110

gct acc gaa atg gtt agg gca gtt gaa gta aac ccg aat aat aga aat       384
Ala Thr Glu Met Val Arg Ala Val Glu Val Asn Pro Asn Asn Arg Asn
            115                 120                 125
```

```
caa gaa gtg tcc ggt gaa tat aca att gag gct tgg aca aag ttt gac     432
Gln Glu Val Ser Gly Glu Tyr Thr Ile Glu Ala Trp Thr Lys Phe Asp
        130                 135                 140

ttt cca gga cga ggt aat act cat tca aac ttc aaa tgg aga tgg tat     480
Phe Pro Gly Arg Gly Asn Thr His Ser Asn Phe Lys Trp Arg Trp Tyr
145                 150                 155                 160

cac ttt gat gga gta gat tgg gat cag tca cgt aag ctg aac aat cga     528
His Phe Asp Gly Val Asp Trp Asp Gln Ser Arg Lys Leu Asn Asn Arg
                165                 170                 175

att tat aaa ttt aga ggt gat gga aaa ggg tgg gat tgg gaa gtc gat     576
Ile Tyr Lys Phe Arg Gly Asp Gly Lys Gly Trp Asp Trp Glu Val Asp
                180                 185                 190

aca gaa aac ggt aac tat gat tac cta atg tat gca gat att gac atg     624
Thr Glu Asn Gly Asn Tyr Asp Tyr Leu Met Tyr Ala Asp Ile Asp Met
                195                 200                 205

gat cac cca gag gta gtg aat gag cta aga aat tgg ggt gtt tgg tat     672
Asp His Pro Glu Val Val Asn Glu Leu Arg Asn Trp Gly Val Trp Tyr
        210                 215                 220

acg aat aca tta ggc ctt gat ggt ttt aga ata gat gca gta aaa cat     720
Thr Asn Thr Leu Gly Leu Asp Gly Phe Arg Ile Asp Ala Val Lys His
225                 230                 235                 240

ata aaa tac agc ttt act cgt gat tgg att aat cat gtt aga agt gca     768
Ile Lys Tyr Ser Phe Thr Arg Asp Trp Ile Asn His Val Arg Ser Ala
                245                 250                 255

act ggc aaa aat atg ttt gcg gtt gcg gaa ttt tgg aaa aat gat tta     816
Thr Gly Lys Asn Met Phe Ala Val Ala Glu Phe Trp Lys Asn Asp Leu
        260                 265                 270

ggt gct att gaa aac tat tta aac aaa aca aac tgg aac cat tca gtc     864
Gly Ala Ile Glu Asn Tyr Leu Asn Lys Thr Asn Trp Asn His Ser Val
        275                 280                 285

ttt gat gtt ccg ctg cac tat aac ctc tat aat gct tca aaa agc gga     912
Phe Asp Val Pro Leu His Tyr Asn Leu Tyr Asn Ala Ser Lys Ser Gly
        290                 295                 300

ggg aat tat gat atg agg caa ata ttt aat ggt aca gtc gtg caa aga     960
Gly Asn Tyr Asp Met Arg Gln Ile Phe Asn Gly Thr Val Val Gln Arg
305                 310                 315                 320

cat cca atg cat gct gtt aca ttt gtt gat aat cat gat tcg caa cct    1008
His Pro Met His Ala Val Thr Phe Val Asp Asn His Asp Ser Gln Pro
                325                 330                 335

gaa gaa gct tta gag tct ttt gtt gaa gaa tgg ttc aaa cca tta gcg    1056
Glu Glu Ala Leu Glu Ser Phe Val Glu Glu Trp Phe Lys Pro Leu Ala
        340                 345                 350

tat gct ttg aca tta aca cgt gaa caa ggc tac cct tct gta ttt tat    1104
Tyr Ala Leu Thr Leu Thr Arg Glu Gln Gly Tyr Pro Ser Val Phe Tyr
        355                 360                 365
```

```
gga gat tat tat ggc att cca acg cat ggt gta cca gcg atg aaa tcg        1152
Gly Asp Tyr Tyr Gly Ile Pro Thr His Gly Val Pro Ala Met Lys Ser
    370             375             380

aaa att gac ccg att cta gaa gcg cgt caa aag tat gca tat gga aga        1200
Lys Ile Asp Pro Ile Leu Glu Ala Arg Gln Lys Tyr Ala Tyr Gly Arg
385             390             395             400

caa aat gac tac tta gac cat cat aat atc atc ggt tgg aca cgt gaa        1248
Gln Asn Asp Tyr Leu Asp His His Asn Ile Ile Gly Trp Thr Arg Glu
            405             410             415

ggg aat aca gca cac ccc aac tcc ggt tta gct act atc atg tcc gat        1296
Gly Asn Thr Ala His Pro Asn Ser Gly Leu Ala Thr Ile Met Ser Asp
            420             425             430

ggg gca gga gga aat aag tgg atg ttt gtt ggg cgt aat aaa gct ggt        1344
Gly Ala Gly Gly Asn Lys Trp Met Phe Val Gly Arg Asn Lys Ala Gly
            435             440             445

caa gtt tgg acc gat atc act gga aat cgt gca ggt act gtt acg att        1392
Gln Val Trp Thr Asp Ile Thr Gly Asn Arg Ala Gly Thr Val Thr Ile
    450             455             460

aat gct gat gga tgg ggt aat ttt tct gta aat gga gga tca gtt tct        1440
Asn Ala Asp Gly Trp Gly Asn Phe Ser Val Asn Gly Gly Ser Val Ser
465             470             475             480

att tgg gta aac aaa                                                     1455
Ile Trp Val Asn Lys
            485
```

<210> 2
<211> 485
<212> PRT
<213> Bacillus sp.

<400> 2

```
    His His Asn Gly Thr Asn Gly Thr Met Met Gln Tyr Phe Glu Trp Tyr
    1               5                   10                  15

    Leu Pro Asn Asp Gly Asn His Trp Asn Arg Leu Arg Ser Asp Ala Ser
                20                  25                  30

    Asn Leu Lys Asp Lys Gly Ile Ser Ala Val Trp Ile Pro Pro Ala Trp
                35                  40                  45

    Lys Gly Ala Ser Gln Asn Asp Val Gly Tyr Gly Ala Tyr Asp Leu Tyr
            50                  55                  60

    Asp Leu Gly Glu Phe Asn Gln Lys Gly Thr Ile Arg Thr Lys Tyr Gly
    65                  70                  75                  80
```

```
Thr Arg Asn Gln Leu Gln Ala Ala Val Asn Ala Leu Lys Ser Asn Gly
                  85              90                  95

Ile Gln Val Tyr Gly Asp Val Val Met Asn His Lys Gly Gly Ala Asp
              100             105             110

Ala Thr Glu Met Val Arg Ala Val Glu Val Asn Pro Asn Asn Arg Asn
          115             120             125

Gln Glu Val Ser Gly Glu Tyr Thr Ile Glu Ala Trp Thr Lys Phe Asp
      130             135             140

Phe Pro Gly Arg Gly Asn Thr His Ser Asn Phe Lys Trp Arg Trp Tyr
145             150             155             160

His Phe Asp Gly Val Asp Trp Asp Gln Ser Arg Lys Leu Asn Asn Arg
              165             170             175

Ile Tyr Lys Phe Arg Gly Asp Gly Lys Gly Trp Asp Trp Glu Val Asp
          180             185             190

Thr Glu Asn Gly Asn Tyr Asp Tyr Leu Met Tyr Ala Asp Ile Asp Met
          195             200             205

Asp His Pro Glu Val Val Asn Glu Leu Arg Asn Trp Gly Val Trp Tyr
    210             215             220

Thr Asn Thr Leu Gly Leu Asp Gly Phe Arg Ile Asp Ala Val Lys His
225             230             235             240

Ile Lys Tyr Ser Phe Thr Arg Asp Trp Ile Asn His Val Arg Ser Ala
              245             250             255

Thr Gly Lys Asn Met Phe Ala Val Ala Glu Phe Trp Lys Asn Asp Leu
          260             265             270

Gly Ala Ile Glu Asn Tyr Leu Asn Lys Thr Asn Trp Asn His Ser Val
          275             280             285

Phe Asp Val Pro Leu His Tyr Asn Leu Tyr Asn Ala Ser Lys Ser Gly
    290             295             300

Gly Asn Tyr Asp Met Arg Gln Ile Phe Asn Gly Thr Val Val Gln Arg
305             310             315             320
```

16

```
His Pro Met His Ala Val Thr Phe Val Asp Asn His Asp Ser Gln Pro
            325                 330                 335

Glu Glu Ala Leu Glu Ser Phe Val Glu Glu Trp Phe Lys Pro Leu Ala
            340                 345                 350

Tyr Ala Leu Thr Leu Thr Arg Glu Gln Gly Tyr Pro Ser Val Phe Tyr
            355                 360                 365

Gly Asp Tyr Tyr Gly Ile Pro Thr His Gly Val Pro Ala Met Lys Ser
        370                 375                 380

Lys Ile Asp Pro Ile Leu Glu Ala Arg Gln Lys Tyr Ala Tyr Gly Arg
385                 390                 395                 400

Gln Asn Asp Tyr Leu Asp His His Asn Ile Ile Gly Trp Thr Arg Glu
                405                 410                 415

Gly Asn Thr Ala His Pro Asn Ser Gly Leu Ala Thr Ile Met Ser Asp
                420                 425                 430

Gly Ala Gly Gly Asn Lys Trp Met Phe Val Gly Arg Asn Lys Ala Gly
            435                 440                 445

Gln Val Trp Thr Asp Ile Thr Gly Asn Arg Ala Gly Thr Val Thr Ile
    450                 455                 460

Asn Ala Asp Gly Trp Gly Asn Phe Ser Val Asn Gly Gly Ser Val Ser
465                 470                 475                 480

Ile Trp Val Asn Lys
                485
```

```
<210> 3
<211> 1860
<212> DNA
<213> Bacillus flavothermus

<220>
<221> CDS
<222> (1)..(1857)

<220>
<221> sig_peptide
<222> (1)..(99)

<220>
<221> mat_peptide
<222> (100)..(1857)
```

<220>
<221> misc_feature
<222> (100)..(1551)
<223> catalysstic domain

<220>
<221> misc_feature
<222> (1552)..(1857)
<223> carbohydrate binding domain

<400> 3

```
atg tcc cta ttc aaa aaa agc ttt ccg tgg att tta tcc cta ctt ctt        48
Met Ser Leu Phe Lys Lys Ser Phe Pro Trp Ile Leu Ser Leu Leu Leu
        -30                 -25                 -20

ttg ttt tcg ttt att gct cct ttt tcc att caa aca gaa aaa gtc cga        96
Leu Phe Ser Phe Ile Ala Pro Phe Ser Ile Gln Thr Glu Lys Val Arg
        -15                 -10                 -5

gct gga agt gtg ccg gta aat ggc aca atg atg caa tat ttc gaa tgg       144
Ala Gly Ser Val Pro Val Asn Gly Thr Met Met Gln Tyr Phe Glu Trp
-1  1               5                   10                  15

tac ctt cca gac gat gga aca cta tgg acg aaa gta gca aat aac gct       192
Tyr Leu Pro Asp Asp Gly Thr Leu Trp Thr Lys Val Ala Asn Asn Ala
            20                  25                  30

caa tct tta gcg aat ctt ggc att act gcc ctt tgg ctt ccc cct gcc       240
Gln Ser Leu Ala Asn Leu Gly Ile Thr Ala Leu Trp Leu Pro Pro Ala
            35                  40                  45

tat aaa gga aca agc agc agt gac gtt gga tat ggc gtt tat gat tta       288
Tyr Lys Gly Thr Ser Ser Ser Asp Val Gly Tyr Gly Val Tyr Asp Leu
        50                  55                  60

tat gac ctt gga gag ttt aat caa aaa gga act gtc cga aca aaa tac       336
Tyr Asp Leu Gly Glu Phe Asn Gln Lys Gly Thr Val Arg Thr Lys Tyr
    65                  70                  75

ggg aca aaa aca caa tat atc caa gca atc caa gcg gcg cat aca gca       384
Gly Thr Lys Thr Gln Tyr Ile Gln Ala Ile Gln Ala Ala His Thr Ala
80                  85                  90                  95

ggg atg caa gta tat gca gat gtc gtc ttt aac cat aaa gcc ggt gca       432
Gly Met Gln Val Tyr Ala Asp Val Val Phe Asn His Lys Ala Gly Ala
                100                 105                 110

gat gga aca gaa cta gtc gat gca gta gaa gta aat cct tct gac cgc       480
Asp Gly Thr Glu Leu Val Asp Ala Val Glu Val Asn Pro Ser Asp Arg
                115                 120                 125

aat caa gaa ata tca gga aca tat caa atc caa gcg tgg aca aaa ttt       528
Asn Gln Glu Ile Ser Gly Thr Tyr Gln Ile Gln Ala Trp Thr Lys Phe
            130                 135                 140

gat ttt cct ggt cgt gga aac acc tat tct agt ttt aaa tgg cgt tgg       576
Asp Phe Pro Gly Arg Gly Asn Thr Tyr Ser Ser Phe Lys Trp Arg Trp
```

```
                145                    150                    155

        tat cat ttc gat gga acg gac tgg gat gag agt aga aaa cta aat cgt      624
        Tyr His Phe Asp Gly Thr Asp Trp Asp Glu Ser Arg Lys Leu Asn Arg
        160                    165                    170                175

        att tac aag ttc cgc ggc acg gga aaa gca tgg gat tgg gaa gta gat      672
        Ile Tyr Lys Phe Arg Gly Thr Gly Lys Ala Trp Asp Trp Glu Val Asp
                        180                    185                190

        aca gaa aac ggg aat tat gac tat ctc atg tat gca gat tta gat atg      720
        Thr Glu Asn Gly Asn Tyr Asp Tyr Leu Met Tyr Ala Asp Leu Asp Met
                        195                    200                205

        gat cat cca gag gtt gta tcc gaa cta aaa aat tgg gga aag tgg tat      768
        Asp His Pro Glu Val Val Ser Glu Leu Lys Asn Trp Gly Lys Trp Tyr
                    210                    215                220

        gta acc aca acc aat atc gac gga ttc cgt ctg gat gca gtg aag cat      816
        Val Thr Thr Thr Asn Ile Asp Gly Phe Arg Leu Asp Ala Val Lys His
            225                    230                    235

        att aaa tat agc ttt ttc ccg gac tgg cta tcg tac gta cga acc caa      864
        Ile Lys Tyr Ser Phe Phe Pro Asp Trp Leu Ser Tyr Val Arg Thr Gln
        240                    245                    250                255

        aca caa aag cct ctt ttt gcc gtt ggg gaa ttt tgg agc tat gac att      912
        Thr Gln Lys Pro Leu Phe Ala Val Gly Glu Phe Trp Ser Tyr Asp Ile
                        260                    265                270

        agc aag ttg cac aac tat att aca aag acg aac ggc tct atg tcc cta      960
        Ser Lys Leu His Asn Tyr Ile Thr Lys Thr Asn Gly Ser Met Ser Leu
                    275                    280                285

        ttc gat gcc ccg ctg cat aac aat ttt tat ata gca tcg aaa tca ggc     1008
        Phe Asp Ala Pro Leu His Asn Asn Phe Tyr Ile Ala Ser Lys Ser Gly
                    290                    295                300

        ggt tat ttt gat atg cgc aca tta ctc aac aac aca ttg atg aaa gat     1056
        Gly Tyr Phe Asp Met Arg Thr Leu Leu Asn Asn Thr Leu Met Lys Asp
            305                    310                    315

        cag cct aca tta gca gtc aca tta gtg gat aat cac gat act gag cca     1104
        Gln Pro Thr Leu Ala Val Thr Leu Val Asp Asn His Asp Thr Glu Pro
        320                    325                    330                335

        ggg caa tct ctg cag tca tgg gtc gag cca tgg ttt aaa ccg tta gct     1152
        Gly Gln Ser Leu Gln Ser Trp Val Glu Pro Trp Phe Lys Pro Leu Ala
                        340                    345                350

        tac gca ttt atc ttg acc cgc caa gaa ggt tat cct tgc gtc ttt tat     1200
        Tyr Ala Phe Ile Leu Thr Arg Gln Glu Gly Tyr Pro Cys Val Phe Tyr
                    355                    360                    365

        gga gat tac tat ggt att cca aaa tac aac att cct gcg ctg aaa agc     1248
        Gly Asp Tyr Tyr Gly Ile Pro Lys Tyr Asn Ile Pro Ala Leu Lys Ser
                    370                    375                    380

        aaa ctt gat ccg ctg tta att gcc aga aga gat tat gcc tat gga aca     1296
        Lys Leu Asp Pro Leu Leu Ile Ala Arg Arg Asp Tyr Ala Tyr Gly Thr
```

385                          390                          395

```
cag cac gac tat att gac agt gcg gat att atc ggt tgg acg cgg gaa    1344
Gln His Asp Tyr Ile Asp Ser Ala Asp Ile Ile Gly Trp Thr Arg Glu
400             405                 410                 415

gga gtg gct gaa aaa gca aat tca gga ctg gct gca ctc att acc gac    1392
Gly Val Ala Glu Lys Ala Asn Ser Gly Leu Ala Ala Leu Ile Thr Asp
                420                 425                 430

ggg cct ggc gga agc aaa tgg atg tat gtt gga aaa caa cac gct ggc    1440
Gly Pro Gly Gly Ser Lys Trp Met Tyr Val Gly Lys Gln His Ala Gly
                435                 440                 445

aaa acg ttt tat gat tta acc ggc aat cga agt gat aca gtg aca atc    1488
Lys Thr Phe Tyr Asp Leu Thr Gly Asn Arg Ser Asp Thr Val Thr Ile
            450                 455                 460

aat gct gat gga tgg gga gaa ttt aaa gtc aat gga ggg tct gta tcc    1536
Asn Ala Asp Gly Trp Gly Glu Phe Lys Val Asn Gly Gly Ser Val Ser
            465                 470                 475

ata tgg gtt cca aaa ata tca acc act tcc caa ata aca ttt act gta    1584
Ile Trp Val Pro Lys Ile Ser Thr Thr Ser Gln Ile Thr Phe Thr Val
480                 485                 490                 495

aat aac gcc aca acc gtt tgg gga caa aat gta tac gtt gtc ggg aat    1632
Asn Asn Ala Thr Thr Val Trp Gly Gln Asn Val Tyr Val Val Gly Asn
                500                 505                 510

att tcg cag ctg ggg aac tgg gat cca gtc cac gca gtt caa atg acg    1680
Ile Ser Gln Leu Gly Asn Trp Asp Pro Val His Ala Val Gln Met Thr
                515                 520                 525

ccg tct tct tat cca aca tgg act gta aca atc cct ctt ctt caa ggg    1728
Pro Ser Ser Tyr Pro Thr Trp Thr Val Thr Ile Pro Leu Leu Gln Gly
                530                 535                 540

caa aac ata caa ttt aaa ttt atc aaa aaa gat tca gct gga aat gtc    1776
Gln Asn Ile Gln Phe Lys Phe Ile Lys Lys Asp Ser Ala Gly Asn Val
        545                 550                 555

att tgg gaa gat ata tcg aat cga aca tac acc gtc cca act gct gca    1824
Ile Trp Glu Asp Ile Ser Asn Arg Thr Tyr Thr Val Pro Thr Ala Ala
560                 565                 570                 575

tcc gga gca tat aca gcc agc tgg aac gtg ccc tag                    1860
Ser Gly Ala Tyr Thr Ala Ser Trp Asn Val Pro
                580                 585
```

<210> 4
<211> 619
<212> PRT
<213> Bacillus flavothermus

<400> 4

```
Met Ser Leu Phe Lys Lys Ser Phe Pro Trp Ile Leu Ser Leu Leu Leu
        -30                 -25                 -20
```

20

```
Leu Phe Ser Phe Ile Ala Pro Phe Ser Ile Gln Thr Glu Lys Val Arg
        -15             -10                 -5

Ala Gly Ser Val Pro Val Asn Gly Thr Met Met Gln Tyr Phe Glu Trp
-1  1           5                   10                      15

Tyr Leu Pro Asp Asp Gly Thr Leu Trp Thr Lys Val Ala Asn Asn Ala
            20                  25                      30

Gln Ser Leu Ala Asn Leu Gly Ile Thr Ala Leu Trp Leu Pro Pro Ala
            35                  40                  45

Tyr Lys Gly Thr Ser Ser Ser Asp Val Gly Tyr Gly Val Tyr Asp Leu
        50                  55                  60

Tyr Asp Leu Gly Glu Phe Asn Gln Lys Gly Thr Val Arg Thr Lys Tyr
    65                  70                  75

Gly Thr Lys Thr Gln Tyr Ile Gln Ala Ile Gln Ala Ala His Thr Ala
80                  85                  90                  95

Gly Met Gln Val Tyr Ala Asp Val Val Phe Asn His Lys Ala Gly Ala
            100                 105                 110

Asp Gly Thr Glu Leu Val Asp Ala Val Glu Val Asn Pro Ser Asp Arg
            115                 120                 125

Asn Gln Glu Ile Ser Gly Thr Tyr Gln Ile Gln Ala Trp Thr Lys Phe
        130                 135                 140

Asp Phe Pro Gly Arg Gly Asn Thr Tyr Ser Ser Phe Lys Trp Arg Trp
    145                 150                 155

Tyr His Phe Asp Gly Thr Asp Trp Asp Glu Ser Arg Lys Leu Asn Arg
160                 165                 170                 175

Ile Tyr Lys Phe Arg Gly Thr Gly Lys Ala Trp Asp Trp Glu Val Asp
            180                 185                 190

Thr Glu Asn Gly Asn Tyr Asp Tyr Leu Met Tyr Ala Asp Leu Asp Met
            195                 200                 205

Asp His Pro Glu Val Val Ser Glu Leu Lys Asn Trp Gly Lys Trp Tyr
        210                 215                 220
```

21

```
Val Thr Thr Thr Asn Ile Asp Gly Phe Arg Leu Asp Ala Val Lys His
    225             230             235

Ile Lys Tyr Ser Phe Phe Pro Asp Trp Leu Ser Tyr Val Arg Thr Gln
240             245             250             255

Thr Gln Lys Pro Leu Phe Ala Val Gly Glu Phe Trp Ser Tyr Asp Ile
                260             265                 270

Ser Lys Leu His Asn Tyr Ile Thr Lys Thr Asn Gly Ser Met Ser Leu
                275             280             285

Phe Asp Ala Pro Leu His Asn Asn Phe Tyr Ile Ala Ser Lys Ser Gly
        290             295             300

Gly Tyr Phe Asp Met Arg Thr Leu Leu Asn Asn Thr Leu Met Lys Asp
    305             310             315

Gln Pro Thr Leu Ala Val Thr Leu Val Asp Asn His Asp Thr Glu Pro
320             325             330             335

Gly Gln Ser Leu Gln Ser Trp Val Glu Pro Trp Phe Lys Pro Leu Ala
                340             345             350

Tyr Ala Phe Ile Leu Thr Arg Gln Glu Gly Tyr Pro Cys Val Phe Tyr
            355             360             365

Gly Asp Tyr Tyr Gly Ile Pro Lys Tyr Asn Ile Pro Ala Leu Lys Ser
        370             375             380

Lys Leu Asp Pro Leu Leu Ile Ala Arg Arg Asp Tyr Ala Tyr Gly Thr
    385             390             395

Gln His Asp Tyr Ile Asp Ser Ala Asp Ile Ile Gly Trp Thr Arg Glu
400             405             410             415

Gly Val Ala Glu Lys Ala Asn Ser Gly Leu Ala Ala Leu Ile Thr Asp
                420             425             430

Gly Pro Gly Gly Ser Lys Trp Met Tyr Val Gly Lys Gln His Ala Gly
            435             440             445

Lys Thr Phe Tyr Asp Leu Thr Gly Asn Arg Ser Asp Thr Val Thr Ile
        450             455             460
```

```
    Asn Ala Asp Gly Trp Gly Glu Phe Lys Val Asn Gly Gly Ser Val Ser
        465                 470                 475

    Ile Trp Val Pro Lys Ile Ser Thr Thr Ser Gln Ile Thr Phe Thr Val
    480                 485                 490                 495

    Asn Asn Ala Thr Thr Val Trp Gly Gln Asn Val Tyr Val Val Gly Asn
                    500                 505                 510

    Ile Ser Gln Leu Gly Asn Trp Asp Pro Val His Ala Val Gln Met Thr
                515                 520                 525

    Pro Ser Ser Tyr Pro Thr Trp Thr Val Thr Ile Pro Leu Leu Gln Gly
                530                 535                 540

    Gln Asn Ile Gln Phe Lys Phe Ile Lys Lys Asp Ser Ala Gly Asn Val
        545                 550                 555

    Ile Trp Glu Asp Ile Ser Asn Arg Thr Tyr Thr Val Pro Thr Ala Ala
    560                 565                 570                 575

    Ser Gly Ala Tyr Thr Ala Ser Trp Asn Val Pro
                580                 585
```

<210> 5
<211> 1920
<212> DNA
<213> Bacillus licheniformis

<220>
<221> CDS
<222> (421)..(1872)

<400> 5

```
cggaagattg gaagtacaaa aataagcaaa agattgtcaa tcatgtcatg agccatgcgg      60

gagacggaaa aatcgtctta atgcacgata tttatgcaac gttcgcagat gctgctgaag     120

agattattaa aaagctgaaa gcaaaaggct atcaattggt aactgtatct cagcttgaag     180

aagtgaagaa gcagagaggc tattgaataa atgagtagaa gcgccatatc ggcgcttttc     240

ttttggaaga aaatataggg aaaatggtac ttgttaaaaa ttcggaatat ttatacaaca     300

tcatatgttt cacattgaaa ggggaggaga atcatgaaac aacaaaaacg gctttacgcc     360

cgattgctga cgctgttatt tgcgctcatc ttcttgctgc ctcattctgc agcagcggcg     420

gca aat ctt aat ggg acg ctg atg cag tat ttt gaa tgg tac atg ccc      468
```

```
      Ala Asn Leu Asn Gly Thr Leu Met Gln Tyr Phe Glu Trp Tyr Met Pro
      1               5                   10                  15


      aat gac ggc caa cat tgg agg cgt ttg caa aac gac tcg gca tat ttg      516
      Asn Asp Gly Gln His Trp Arg Arg Leu Gln Asn Asp Ser Ala Tyr Leu
                      20                  25                  30


      gct gaa cac ggt att act gcc gtc tgg att ccc ccg gca tat aag gga      564
      Ala Glu His Gly Ile Thr Ala Val Trp Ile Pro Pro Ala Tyr Lys Gly
                  35                  40                  45


      acg agc caa gcg gat gtg ggc tac ggt gct tac gac ctt tat gat tta      612
      Thr Ser Gln Ala Asp Val Gly Tyr Gly Ala Tyr Asp Leu Tyr Asp Leu
              50                  55                  60


      ggg gag ttt cat caa aaa ggg acg gtt cgg aca aag tac ggc aca aaa      660
      Gly Glu Phe His Gln Lys Gly Thr Val Arg Thr Lys Tyr Gly Thr Lys
      65                  70                  75                  80


      gga gag ctg caa tct gcg atc aaa agt ctt cat tcc cgc gac att aac      708
      Gly Glu Leu Gln Ser Ala Ile Lys Ser Leu His Ser Arg Asp Ile Asn
                      85                  90                  95


      gtt tac ggg gat gtg gtc atc aac cac aaa ggc ggc gct gat gcg acc      756
      Val Tyr Gly Asp Val Val Ile Asn His Lys Gly Gly Ala Asp Ala Thr
                      100                 105                 110


      gaa gat gta acc gcg gtt gaa gtc gat ccc gct gac cgc aac cgc gta      804
      Glu Asp Val Thr Ala Val Glu Val Asp Pro Ala Asp Arg Asn Arg Val
                  115                 120                 125


      att tca gga gaa cac cta att aaa gcc tgg aca cat ttt cat ttt ccg      852
      Ile Ser Gly Glu His Leu Ile Lys Ala Trp Thr His Phe His Phe Pro
              130                 135                 140


      ggg cgc ggc agc aca tac agc gat ttt aaa tgg cat tgg tac cat ttt      900
      Gly Arg Gly Ser Thr Tyr Ser Asp Phe Lys Trp His Trp Tyr His Phe
      145                 150                 155                 160


      gac gga acc gat tgg gac gag tcc cga aag ctg aac cgc atc tat aag      948
      Asp Gly Thr Asp Trp Asp Glu Ser Arg Lys Leu Asn Arg Ile Tyr Lys
                      165                 170                 175


      ttt caa gga aag gct tgg gat tgg gaa gtt tcc aat gaa aac ggc aac      996
      Phe Gln Gly Lys Ala Trp Asp Trp Glu Val Ser Asn Glu Asn Gly Asn
                      180                 185                 190


      tat gat tat ttg atg tat gcc gac atc gat tat gac cat cct gat gtc      1044
      Tyr Asp Tyr Leu Met Tyr Ala Asp Ile Asp Tyr Asp His Pro Asp Val
                  195                 200                 205


      gca gca gaa att aag aga tgg ggc act tgg tat gcc aat gaa ctg caa      1092
      Ala Ala Glu Ile Lys Arg Trp Gly Thr Trp Tyr Ala Asn Glu Leu Gln
              210                 215                 220


      ttg gac ggt ttc cgt ctt gat gct gtc aaa cac att aaa ttt tct ttt      1140
      Leu Asp Gly Phe Arg Leu Asp Ala Val Lys His Ile Lys Phe Ser Phe
      225                 230                 235                 240


      ttg cgg gat tgg gtt aat cat gtc agg gaa aaa acg ggg aag gaa atg      1188
```

```
Leu Arg Asp Trp Val Asn His Val Arg Glu Lys Thr Gly Lys Glu Met
                245                 250                 255

ttt acg gta gct gaa tat tgg cag aat gac ttg ggc gcg ctg gaa aac    1236
Phe Thr Val Ala Glu Tyr Trp Gln Asn Asp Leu Gly Ala Leu Glu Asn
            260                 265                 270

tat ttg aac aaa aca aat ttt aat cat tca gtg ttt gac gtg ccg ctt    1284
Tyr Leu Asn Lys Thr Asn Phe Asn His Ser Val Phe Asp Val Pro Leu
            275                 280                 285

cat tat cag ttc cat gct gca tcg aca cag gga ggc ggc tat gat atg    1332
His Tyr Gln Phe His Ala Ala Ser Thr Gln Gly Gly Gly Tyr Asp Met
        290                 295                 300

agg aaa ttg ctg aac ggt acg gtc gtt tcc aag cat ccg ttg aaa tcg    1380
Arg Lys Leu Leu Asn Gly Thr Val Val Ser Lys His Pro Leu Lys Ser
305                 310                 315                 320

gtt aca ttt gtc gat aac cat gat aca cag ccg ggg caa tcg ctt gag    1428
Val Thr Phe Val Asp Asn His Asp Thr Gln Pro Gly Gln Ser Leu Glu
                325                 330                 335

tcg act gtc caa aca tgg ttt aag ccg ctt gct tac gct ttt att ctc    1476
Ser Thr Val Gln Thr Trp Phe Lys Pro Leu Ala Tyr Ala Phe Ile Leu
            340                 345                 350

aca agg gaa tct gga tac cct cag gtt ttc tac ggg gat atg tac ggg    1524
Thr Arg Glu Ser Gly Tyr Pro Gln Val Phe Tyr Gly Asp Met Tyr Gly
            355                 360                 365

acg aaa gga gac tcc cag cgc gaa att cct gcc ttg aaa cac aaa att    1572
Thr Lys Gly Asp Ser Gln Arg Glu Ile Pro Ala Leu Lys His Lys Ile
        370                 375                 380

gaa ccg atc tta aaa gcg aga aaa cag tat gcg tac gga gca cag cat    1620
Glu Pro Ile Leu Lys Ala Arg Lys Gln Tyr Ala Tyr Gly Ala Gln His
385                 390                 395                 400

gat tat ttc gac cac cat gac att gtc ggc tgg aca agg gaa ggc gac    1668
Asp Tyr Phe Asp His His Asp Ile Val Gly Trp Thr Arg Glu Gly Asp
                405                 410                 415

agc tcg gtt gca aat tca ggt ttg gcg gca tta ata aca gac gga ccc    1716
Ser Ser Val Ala Asn Ser Gly Leu Ala Ala Leu Ile Thr Asp Gly Pro
            420                 425                 430

ggt ggg gca aag cga atg tat gtc ggc cgg caa aac gcc ggt gag aca    1764
Gly Gly Ala Lys Arg Met Tyr Val Gly Arg Gln Asn Ala Gly Glu Thr
        435                 440                 445

tgg cat gac att acc gga aac cgt tcg gag ccg gtt gtc atc aat tcg    1812
Trp His Asp Ile Thr Gly Asn Arg Ser Glu Pro Val Val Ile Asn Ser
    450                 455                 460

gaa ggc tgg gga gag ttt cac gta aac ggc ggg tcg gtt tca att tat    1860
Glu Gly Trp Gly Glu Phe His Val Asn Gly Gly Ser Val Ser Ile Tyr
465                 470                 475                 480

gtt caa aga tag aagagcagag aggacggatt tcctgaagga aatccgtttt        1912
Val Gln Arg
```

25

Val Gln Arg


tttatttt                                                                    1920

<210> 6
<211> 483
<212> PRT
<213> Bacillus licheniformis


<400> 6


Ala Asn Leu Asn Gly Thr Leu Met Gln Tyr Phe Glu Trp Tyr Met Pro
1               5                   10                  15


Asn Asp Gly Gln His Trp Arg Arg Leu Gln Asn Asp Ser Ala Tyr Leu
            20                  25                  30


Ala Glu His Gly Ile Thr Ala Val Trp Ile Pro Pro Ala Tyr Lys Gly
            35                  40                  45


Thr Ser Gln Ala Asp Val Gly Tyr Gly Ala Tyr Asp Leu Tyr Asp Leu
        50                  55                  60


Gly Glu Phe His Gln Lys Gly Thr Val Arg Thr Lys Tyr Gly Thr Lys
65                  70                  75                  80


Gly Glu Leu Gln Ser Ala Ile Lys Ser Leu His Ser Arg Asp Ile Asn
                85                  90                  95


Val Tyr Gly Asp Val Val Ile Asn His Lys Gly Gly Ala Asp Ala Thr
            100                 105                 110


Glu Asp Val Thr Ala Val Glu Val Asp Pro Ala Asp Arg Asn Arg Val
            115                 120                 125


Ile Ser Gly Glu His Leu Ile Lys Ala Trp Thr His Phe His Phe Pro
    130                 135                 140


Gly Arg Gly Ser Thr Tyr Ser Asp Phe Lys Trp His Trp Tyr His Phe
145                 150                 155                 160


Asp Gly Thr Asp Trp Asp Glu Ser Arg Lys Leu Asn Arg Ile Tyr Lys
                165                 170                 175


Phe Gln Gly Lys Ala Trp Asp Trp Glu Val Ser Asn Glu Asn Gly Asn
                180                 185                 190

```
Tyr Asp Tyr Leu Met Tyr Ala Asp Ile Asp Tyr Asp His Pro Asp Val
        195             200             205

Ala Ala Glu Ile Lys Arg Trp Gly Thr Trp Tyr Ala Asn Glu Leu Gln
    210             215             220

Leu Asp Gly Phe Arg Leu Asp Ala Val Lys His Ile Lys Phe Ser Phe
225             230             235             240

Leu Arg Asp Trp Val Asn His Val Arg Glu Lys Thr Gly Lys Glu Met
            245             250             255

Phe Thr Val Ala Glu Tyr Trp Gln Asn Asp Leu Gly Ala Leu Glu Asn
            260             265             270

Tyr Leu Asn Lys Thr Asn Phe Asn His Ser Val Phe Asp Val Pro Leu
        275             280             285

His Tyr Gln Phe His Ala Ala Ser Thr Gln Gly Gly Gly Tyr Asp Met
    290             295             300

Arg Lys Leu Leu Asn Gly Thr Val Val Ser Lys His Pro Leu Lys Ser
305             310             315             320

Val Thr Phe Val Asp Asn His Asp Thr Gln Pro Gly Gln Ser Leu Glu
            325             330             335

Ser Thr Val Gln Thr Trp Phe Lys Pro Leu Ala Tyr Ala Phe Ile Leu
        340             345             350

Thr Arg Glu Ser Gly Tyr Pro Gln Val Phe Tyr Gly Asp Met Tyr Gly
        355             360             365

Thr Lys Gly Asp Ser Gln Arg Glu Ile Pro Ala Leu Lys His Lys Ile
    370             375             380

Glu Pro Ile Leu Lys Ala Arg Lys Gln Tyr Ala Tyr Gly Ala Gln His
385             390             395             400

Asp Tyr Phe Asp His His Asp Ile Val Gly Trp Thr Arg Glu Gly Asp
            405             410             415

Ser Ser Val Ala Asn Ser Gly Leu Ala Ala Leu Ile Thr Asp Gly Pro
        420             425             430
```

```
Gly Gly Ala Lys Arg Met Tyr Val Gly Arg Gln Asn Ala Gly Glu Thr
        435             440                 445

Trp His Asp Ile Thr Gly Asn Arg Ser Glu Pro Val Val Ile Asn Ser
    450             455                 460

Glu Gly Trp Gly Glu Phe His Val Asn Gly Gly Ser Val Ser Ile Tyr
465             470                 475                 480

Val Gln Arg
```

**Description**

**Field of the Invention**

The present invention relates to improved methods of preventing, removing, reducing, or disrupting biofilm formation on a surface.

**Description of the Related Art**

Biofilms are biological films that develop and persist at the surfaces of biotic or abiotic objects in aqueous environments from the adsorption of microbial cells onto the solid surfaces. This adsorption can provide a competitive advantage for the microorganisms since they can reproduce, are accessible to a wider variety of nutrients and oxygen conditions, are not washed away, and are less sensitive to antimicrobial agents. The formation of the biofilm is also accompanied by the production of exo-polymeric materials (polysaccharides, polyuronic acids, alginates, glycoproteins, and proteins) which together with the cells form thick layers of differentiated structures separated by water-filled spaces. The resident microorganisms may be individual species of microbial cells or mixed communities of microbial cells, which may include aerobic and anaerobic bacteria, algae, protozoa, and fungi. Thus, the biofilm is a complex assembly of living microorganisms embedded in an organic structure composed of one or more matrix polymers which are secreted by the resident microorganisms.

Biofilms can develop into macroscopic structures several millimeters or centimeters in thickness and cover large surface areas. These formations can play a role in restricting or entirely blocking flow in plumbing systems, decreasing heat transfer in heat exchangers, or causing pathogenic problems in municipal water supplies, food processing, medical devices (e.g., catheters, orthopedic devices, implants, endoscopes). Moreover, biofilms often decrease the life of materials through corrosive action mediated by the embedded microorganisms. This biological fouling is a serious economic problem in industrial water process systems, pulp and paper production processes, cooling water systems, injection wells for oil recovery, cooling towers, porous media (sand and soil), marine environments, and air conditioning systems, and any closed water recirculation system. Biofilms are also a severe problem in medical science and industry causing dental plaque, infections (Costerton et al., 1999, Science 284: 1318-1322), contaminated endoscopes and contact lenses, prosthetic device colonisation and biofilm formation on medical implants.

The removal or prevention of biofilm traditionally requires the use of dispersants, surfactants, detergents, enzyme formulations, anti-microbials, biocides, boil-out procedures, and/or corrosive chemicals, e.g., base. Procedures for using these measures are well known in the art. For example, removal of biofilm built-up in a paper machine in the pulp and paper industry traditionally requires a deposit control program including proper housekeeping to keep surfaces free of splashed stock, anti-microbial treatment of fresh water and additives, the use of biocides to reduce microbiological growth on the machine, and scheduled boil-outs to remove the deposits that do form.

Bacteria growing in biofilms are more resistant to antibiotics and disinfectants than planktonic cells and the resistance increases with the age of the biofilm. Bacterial biofilm also exhibits increased physical resistance towards desiccation, extreme temperatures or light. As mentioned, biofilm formation causes industrial, environmental and medical problems and the difficulties in cleaning and disinfection of bacterial biofilm with chemicals is a major concern in many industries. Furthermore, the trend towards milder disinfection and cleaning compositions to reduce their environmental impact may increase the insufficient cleaning of surfaces covered with biofilm. WO00/22103, WO99/20239, WO98/26807 and WO01/98214 disclose Bacillus alkaline alpha-amylases and their use for the enzymatic treatment of biofilm. WO01/66712 discloses alpha-amylase variants.

It is an object of the present invention to provide improved methods for preventing or removing biofilm present on a surface.

**Brief Description of the Drawing**

Fig. 1 shows a comparison of the percentage (%) of total hydrolysis of raw wheat starch for three different alpha-amylases.
Fig. 2 is a chromatogram comparing 1) Alpha-amylase A, 2) detergent alone 3) Alpha-amylase C biofilm removal.

**Summary of the Invention**

The present invention relates to a method for preventing, removing, reducing, or disrupt biofilm formation on a surface, comprising contacting the surface with alpha-amylase derived from a bacterium, where said alpha-amylase has alpha-amylase activity and an amino acid sequence having at least 80% identity with SEQ ID NO: 2, and wherein the alpha-amylase is selected from the group consisting of:

a) an alpha-amylase comprising an amino acid sequence having a deletion in positions D183 and/or G184 using SEQ ID NO:2 for numbering;
b) an alpha-amylase comprising an amino acid sequence having deletions in positions D183+G184 using SEQ ID NO:2 for numbering; and
c) an alpha-amylase comprising an amino acid sequence having one or more of substitutions selected from the group consisting of: R118K, N195F, R320K, and R458K, and having a deletion in positions D183 and/or G184 using SEQ ID NO:2 for numbering.

The invention further relates to use of such alpha-amylases for prevention or removal of biofilm from surfaces.

The term "surface" is defined herein as any surface which may be covered by biofilm or is prone to biofilm formation. Examples of surfaces may be any hard surface such as metal, plastics, rubber, board, glass, wood, paper, concrete, rock, marble, gypsum and ceramic materials, which optionally are coated, for example, with paint or enamel; any soft surface such as fibers of any kind (e.g., yarns, textiles, vegetable fibers, rock wool, and hair); or any porous surfaces; skin (human or animal); keratinous materials (e.g., nails); and internal organs (e.g., lungs). The hard surface can be present as a part of a cooling tower, water treatment plant, water tanks, dairy, food processing plant, chemical or pharmaceutical process plant, or medical device (e.g., catheters, orthopedic devices, implants). The porous surface can be present in a filter, e.g., a membrane filter.

The term "effective amount" is defined herein as the amount of one or more alpha-amylases that is sufficient to degrade a microbially-produced biofilm comprising alpha-1,4 glucosidic linkages. The effective amount of the one or more alpha-amylase will depend on factors including: the alpha-amylase(s) in question, whether the aim is preventing, removing, or reducing biofilms present on a surface, the period of time desirable for, e.g., degrading a microbially-produced biofilm. High amounts/concentrations of enzyme(s) will in general require shorter times of treatment, while low amounts/concentrations longer times. Further, for instance, preventing biofilm on a surface prone to biofilm formation will in general require lower amounts/concentrations of enzyme(s) than the actual removal of biofilm from a corresponding contaminated surface. However, typical effective usage levels are between 0.005 to 500 mg of alpha-amylase protein per L biofilm control solution, preferably between 0.01 - 100 mg of enzyme protein per L biofilm control solution. The term "biofilm control solution" refers to a solution used according to the invention for preventing, removing, reducing or disrupting biofilm present on a surface. The method of the invention may result in $10\text{-}10^8$-fold, preferably $10^3\text{-}10^6$-fold biofilm reduction in terms of average plate count under the conditions indicated in Example 4 below.

## Detailed Description of the Invention

The present invention relates to improved methods of preventing, removing, or reducing biofilms present on a surface, comprising contacting the surface with an effective amount of an alpha-amylase as defined below. The methods of the present invention may be used to prevent, remove, reduce, or disrupt biofilm formation on a surface. One of ordinary skill in the art will recognize that such methods may be employed at different stages of biofim formation.

By using an alpha-amylase in an effective amount of surfaces improved biofilm prevention and/or removal is obtained, especially in those instances where some of the microbes present in the biofilm produce alpha-1,4 linked glucose polysaccharides such as amylose, amylopectin, mixtures of these two polysaccharides (i.e., starch), and glycogen.

In the first aspect the invention relates to a method for preventing or removing biofilm on a surface, comprising contacting the surface with an alpha-amylase derived from a bacterium. In a preferred embodiment the bacterial alpha-amylase is derived from a strain of *Bacillus.*

### Alpha-Amylase

The alpha-amylase used according to the invention is derived from a bacterium, preferably derived from a strain of *Bacillus* sp., especially selected from the group consisting of: the AA560 alpha-amylase disclosed as SEQ ID NO: 2 in WO 00/60060 (SEQ ID NO: 2 herein), the *Bacillus flavothermus* disclosed in US patent application no. 10/877,847, *Bacillus* sp. alpha-amylases disclosed in WO 95/26397, alpha-amylases derived from *Bacillus* sp. NCIB 12289, NCIB 12512, NCIB 12513, DSM 9375, DSMZ no. 12649, KSM AP1378 (WO 97/00324), KSM K36 or KSM K38 (EP 1,022,334), and the #707 alpha-amylase disclosed by Tsukamoto et al., Biochemical and Biophysical Research Communications, 151 (1988), pp. 25-31.

In a preferred specific embodiment of the invention the alpha-amylase is the AA560 alpha-amylase shown in SEQ ID NO: 2 herein and/or the AMY1048 alpha-amylase shown in SEQ ID NO: 4 herein, or an alpha-amylase having a degree of identity of at least 60%, preferably at least 70%, more preferred at least 80%, even more preferred at least 90%, such as at least 95%, at least 96%, at least 97%, at least 98% or at least 99% to any of the sequences shown in SEQ ID NOS: 2 or 4 herein.

In a preferred embodiment the alpha-amylase used is a variant of the parent alpha-amylase disclosed in SEQ ID NO: 2 herein having a deletion in positions D183 and/or G184, preferably wherein said alpha-amylase variant further has a substitution in or corresponding to position N195F, especially wherein the parent alpha-amylase has one or more of the following deletions/substitutions in SEQ ID NO: 2 herein: Delta (R81-G182); Delta (D183-G184); Delta (D183-G184)+N195F; R181Q+N445Q+K446N; Delta (D183-G184)+R181Q, Delta (D183-G184) and one or more of the following substitutions: R118K, N195F, R320K, R458K, especially wherein said parent alpha-amylase has the following mutations:

Delta (D183+G184)+R118K+N195F+R320K+R458K (i.e., in SEQ ID NO: 2 herein).

In another preferred embodiment the alpha-amylase is the AA560 alpha-amylase shown in SEQ ID NO: 2 or a variant thereof further comprising one or more of the following substitutions: M9L, M202L, V214T, M323T, M382Y or M9L, M202L, V214T, M323T and E345R.

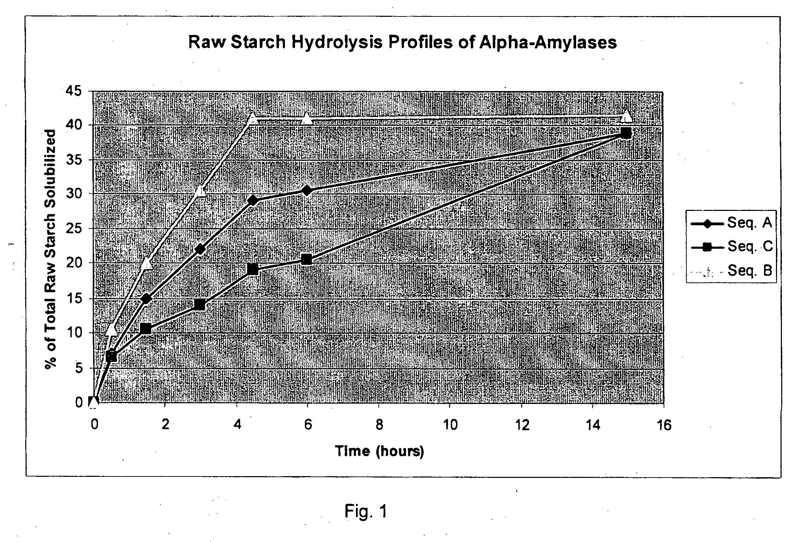

In a preferred embodiment the alpha-amylase has a percentage (%) of hydrolyzed raw starch that is higher than 15, preferably 25, especially 35, after 5 hours at 40°C, 3 mg enzyme protein per g starch, pH 8.0 (See Example 2 and Fig. 1).

In another preferred embodiment the alpha-amylase comprises Asn-Gly-Thr-Met-Met-Gln-Tyr-Phe-Glu-Trp in its N-terminal amino acid region. Examples of such alpha-amylase include Alpha-Amylase A and Alpha-Amylase B used in Example 2.

In an embodiment the alpha-amylase is derived from a strain of *Bacillus licheniformis* with the sequence shown as SEQ ID NO: 6 herein, or an alpha-amylase having a degree of identity of at least 60%, preferably at least 70%, more preferred at least 80%, even more preferred at least 90%, such as at least 95%, at least 96%, at least 97%, at least 98% or at least 99% to any of the sequences shown in SEQ ID NO: 6 herein, preferably with a substitution in a position corresponding to position M197, preferably M197L, T, I, N, D, Q, E,P,W, especially M197L or T.

Commercially available alpha-amylase products or products comprising alpha amylases include product sold under the following trade names: STAINZYME™, DURAMYL™ (Novozymes A/S, Denmark), BIOAMYLASE D(G), BIOAMYLASE™ L (Biocon India Ltd.), KEMZYM™ AT 9000(Biozym Ges. m.b.H, Austria), PURASTAR™ ST, PURASTAR™ HPAmL, PURAFECT™ OxAm, RAPIDASE™ TEX (Genencor Int. Inc, USA), KAM (KAO, Japan).

In a preferred embodiment, surfaces prone to biofilm formation may be subjected to the methods of the present invention as a preventative measure prior to any biofilm formation so no biofilm forms. Alternatively, at the first indication of biofim formation, the methods may be used to prevent further formation and to remove the biofim that has deposited on a surface. Furthermore, in situations where there is a heavy build-up of biofilm on a surface, the methods may be used to reduce the level of biofilm or to remove it partially or completely.

A biofilm may comprise an integrated community of one or two or more microorganisms or predominantly a specific microorganism (Palmer and White, 1997, Trends in Microbiology 5: 435-440; Costerton et al., 1987, Annual Reviews of Microbiology 41: 435-464; Mueller, 1994, TAPPI Proceedings, 1994 Biological Sciences Symposium 195-201). In the methods of the present invention, the one or more microorganisms may be any microorganism involved in biofilm formation including, but not limited to, aerobic bacteria or anaerobic bacteria (Gram positive and Gram negative), fungi (yeast or filamentous fungus), algae, and/or protozoa. Contemplated bacteria include bacteria selected from the group consisting of. *Pseudomonas* spp. including *Pseudomonas aeruginosa, Azotobacter vinelandii, Escherichia coli, Corynebacterium. diphteriae, Clostridium botulinum, Streptococcus spp, Acetobacter, Leuconostoc, Betabacterium, Pneumococci, Mycobacterium tuberculosis, Aeromonas, Burkholderie, Flavobacterium, Salmonella, Staphylococcus*.

In a preferred embodiment, the microorganism is an aerobic bacterium. In a more preferred embodiment, the aerobic bacterium is an *Aeromonas* strain. In another more preferred embodiment, the aerobic bacterium is a *Burkholderie* strain. In another more preferred embodiment, the aerobic bacterium is a *Flavobacterium* strain. In another more preferred embodiment, the aerobic bacterium is a *Microbacterium* strain. In another more preferred embodiment, the aerobic bacterium is a *Pseudomonas* strain. In another more preferred embodiment, the aerobic bacterium is a *Salmonella* strain. In another more preferred embodiment, the aerobic bacterium is a *Staphylococcus* strain. In another more preferred embodiment, the aerobic bacterium is from the family *Enterobacteriaceae* (including *e.g., Escherichia coli*).

In a most preferred embodiment, the aerobic bacterium is *Burkholderie cepacia.* In another most preferred embodiment, the aerobic bacterium is a *Microbacterium imperiale* or *Mycobacterium tuberculosis.* In another most preferred embodiment, the aerobic bacterium is *Pseudomonas aeruginosa.* In another most preferred embodiment, the aerobic bacterium is *Pseudomonas fluorescens.* In another most preferred embodiment, the aerobic bacterium is *Pseudomonas oleovorans.* In another most preferred embodiment, the aerobic bacterium is *Pseudomonas pseudoalcaligenes.* In another most preferred embodiment, the aerobic bacterium is *Salmonella enteritidis.* In another most preferred embodiment, the aerobic bacterium is *Staphylococcus aureus.* In another most preferred embodiment, the aerobic bacterium is *Staphylococcus epidermidis.*

In another preferred embodiment, the microorganism is an anaerobic bacteria. In another more preferred embodiment, the anaerobic bacterium is a *Desulfovibrio* strain. In another most preferred embodiment, the anaerobic bacterium is *Desulfovibrio desulfuricans.*

In another preferred embodiment, the microorganism is a fungus such as a yeast or filamentous fungus. In another more preferred embodiment, the yeast is a *Candida* strain. In another most preferred embodiment, the yeast is *Candida albicans.*

As mentioned above the treatment time for preventing or removing biofilm will depend on the dosage of the alpha-amylase, and the level of biofilm on the surface or prone to the area in question, but should preferably be adapted to the time normally used for conventional treatment of biofilm with antibiotics, biocides, bactericides, fungicides, bleaching agents, surfactants, caustic, and/or biopolymer degrading agents. Consequently, the dosage of the alpha-amylase may be adjusted according to the time period used during conventional treatments. However, where the alpha-amylase treatment is a separate step in the processing, the dosage of the alpha-amylase used will depend on the time period desired to accomplish the treatment.

In terms of alpha-amylase activity, the appropriate dosage of alpha-amylase for preventing or removing biofilms will depend on the amount of biofilm on the surface or prone to the area in question. The skilled person may determine a suitable alpha-amylase unit dosage. The dosage may be expressed in alpha-amylase units. Alpha-amylase units may be determined as "KNU", using the assay described below in the "Materials & Methods"-section. Biofilm contaminated or prone areas are preferably treated for between 1 minute and 2 days, preferably between 10 minutes and 1 day, preferably between 1 hour and 15 hours, more preferably less that 10 hours, with an alpha-amylase dosage of between 0.005 to 500 mg of alpha-amylase protein per L biofilm control solution, preferably between 0.01 to 100 mg of alpha-amylase protein per L biofilm control solution.

The alpha-amylase may be part of a composition to be used in the methods of the present invention. The composition may be in any form suitable for the use in question, e.g., in the form of a dry powder, agglomerated powder, or granulate, in particular a non-dusting granulate, liquid, in particular a stabilized liquid, or protected alpha-amylase. Granulates and agglomerated powders may be prepared by conventional methods, e.g., by spraying the alpha-amylase onto a carrier in a fluid-bed granulator. The carrier may consist of particulate cores having a suitable particle size. The carrier may be soluble or insoluble, e.g., a salt (such as sodium chloride or sodium sulfate), sugar (such as sucrose or lactose), sugar alcohol (such as sorbitol), or starch. The alpha-amylase may be contained in slow-release formulations. Methods for preparing slow-release formulations are well known in the art. Liquid alpha-amylase preparations may, for instance, be stabilized by adding nutritionally acceptable stabilizers such as a sugar, sugar alcohol, or another polyol, and/or lactic acid or another organic acid according to established methods.

The composition may be augmented with one or more agents for preventing or removing the formation of the biofilm. These agents may include, but are not limited to, dispersants, surfactants, detergents, other enzymes, anti-microbials, and biocides.

In a preferred embodiment, the agent is a surfactant. The surfactant may be a non-ionic including semi-polar and/or anionic and/or cationic and/or zwitterionic surfactant.

Anionic surfactants contemplated include linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

Non-ionic surfactants contemplated include alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The surfactants may be present at a level of from 0.1 % to 60% by weight of the enzyme biofilm removal composition.

In a more preferred embodiment, the surfactant is sodium dodecyl sulfate, quaternary ammonium compounds, alkyl pyridinium iodides, Tween 80, Tween, 85, Triton X-100, Brij 56, biological surfactants, rhamnolipid, surfactin, visconsin, or sulfonates.

The formation of biofilm is generally accompanied by the production of exo-polymeric materials (polysaccharides, polyuronic acids, alginates, glycoproteins, and proteins) which together with the cells form thick layers of differentiated structures separated by water-filled spaces (McEldowney and Fletcher, 1986, Journal of General Microbiology 132: 513-523; Sutherland, Surface Carbohydrates of the Prokaryotic Cell, Academic Press, New York, 1977, pp. 27-96). In the methods of the present invention, the alpha-amylase composition may further comprise one or more other enzymes capable of degrading the exo-polymeric materials such as polysaccharides, polyuronic acids, alginates, glycoproteins, and proteins.

Other enzyme activities

In a preferred embodiment, the one or more other enzymes may be selected from the group consisting of an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, oxididases, including carbohydrate oxidases, peroxidases, laccase, lipase, mannosidase, pectinolytic enzyme, peptidoglutaminase, phytase, polyphenoloxidase, proteolytic enzyme, ribonuclease, transglutaminase, or xylanase.

The other enzyme(s) may be selected according to the properties of the specific biofilm which is to be removed, or a combination of several enzymes having different enzyme activities may be used.

In a preferred embodiment, the other enzyme is selected from the group consisting of 1,2-1,3-alpha-D-mannan mannohydrolase, 1,3-beta-D-xylan xylanohydrolase, 1,3-beta-D-glucan glucanohydrolase, 1,3(1,3;1,4)-alpha-D-glucan 3-glucanohydrolase, 1,3(1,3;1,4)-beta-D-glucan 3(4)-glucanohydrolase, 1,3-1,4-alpha-D-glucan 4-glucanohydrolase, 1,4-alpha-D-glucan glucanehydrolase, 1,4-alpha-D-glucan glucohydrolase, 1,4-(1,3:1,4)-beta-D-glucan 4-glucanohydrolase, 1,4-beta-D-glucan glucohydrolase, 1,4-beta-D-xylan xylanohydrolase, 1,4-beta-D-mannan mannanohydrolase, 1,5-alpha-L-arabinan 1,5-alpha-L-arabinanohydrolase, 1,4-alpha-D-glucan maltohydrolase, 1,6-alpha-D-glucan 6-glucanohydrolase, 2,6-beta-D-fructan fructanohydrolase, alpha-Dextrin 6-glucanohydrolase, alpha-D-galactoside galactohydrolase, alpha-D-glucoside glucohydrolase, alpha-D-mannoside mannohydrolase, acylneuraminyl hydrolase, *Aerobacter*-capsular-polysaccharide galactohydrolase, beta-D-fructofuranoside fructohydrolase, beta-D-fucoside fucohydrolase, beta-D-fructan fructohydrolase, beta-D-galactoside galactohydrolase, beta-D-glucoside glucohydrolase, beta-D-glucuronoside glucuronosohydrolase, beta-D-mannoside mannohydrolase, beta-N-acetyl-D-hexosaminide N-acetylhexosamino hydrolase, cellulose-sulfate sulfohydrolase, collagenase, dextrin 6-alpha-D-glucanohydrolase, glycoprotein-phosphatidylinositol phosphatidohydrolase, hyaluronate 4-glycanohydrolase, hyaluronoglucuronidase, pectin pectylhydrolase, peptidoglycan N-acetylmuramoylhydrolase, phosphatidylcholine 2-acylhydrolase, phosphatidylcholine 1-acylhydrolase, poly(1,4-alpha-D-galacturonide) galacturonohydrolase, poly(1,4-(N-acetyl-beta-D-glucosaminide))-glycanohydrolase, sucrose alpha-glucosidase, triacylglycerol acylhydrolase, and triacylglycerol protein-acylhydrolase.

Proteolytic enzyme

The other enzyme may be any enzyme having proteolytic activity under the actual process conditions. Thus, the enzyme may be a proteolytic enzyme of plant origin, e.g., papain, bromelain, ficin, or of animal origin, e.g., trypsin and chymotrypsin, or of microbial origin, i.e., bacterial, yeast, or filamentous fungal. It is understood that any mixture of various proteolytic enzyme may be applicable in the process of the invention.

In another preferred embodiment, the other enzyme is a proteolytic enzyme such as a serine protease, a metalloprotease, or an aspartate protease.

A sub-group of the serine proteases are commonly designated as subtilisins. A subtilisin is a serine protease produced by Gram-positive bacteria or fungi. The amino acid sequence of a number of subtilisins have been determined, including at least six subtilisins from *Bacillus* strains, namely, subtilisin 168, subtilisin BPN, subtilisin Carlsberg, subtilisin DY, subtilisin amylosacchariticus, and mesentericopeptidase, one subtilisin from an actinomycetales, thermitase from *Thermoactinomyces vulgaris,* and one fungal subtilisin, proteinase K from *Tritirachium album.* A further subgroup of the subtilisins, subtilases, has been recognised more recently. Subtilases are described as highly alkaline subtilisins and comprise enzymes such as subtilisin PB92 (MAXACAL®, Gist-Brocades NV), subtilisin 309 (SAVINASE®, Novozymes A/S), and subtilisin 147 (ESPERASE®, Novozymes A/S).

A "subtilisin variant or mutated subtilisin protease" is defined herein as a subtilisin that has been produced by an organism which is expressing a mutant gene derived from a parent microorganism which possessed an original or parent gene and which produced a corresponding parent enzyme, the parent gene having been mutated in order to produce the mutant gene from which said mutated subtilisin protease is produced when expressed in a suitable host. These mentioned subtilisins and variants thereof constitute a preferred class of proteases which are useful in the method of the invention. An example of a useful subtilisin variant is a variant of subtilisin 309 (SAVINASE®) wherein, in position 195, glycine is substituted by phenylalanine (G195F or [195]Gly to [195]Phe).

Commercially available proteases may be used in the methods of the present invention. Examples of such commercial proteases are ALCALASE® (produced by submerged fermentation of a strain of *Bacillus licheniformis),* ESPERASE® (produced by submerged fermentation of an alkalophilic species of *Bacillus),* RENNILASE® (produced by submerged fermentation of a non-pathogenic strain of *Mucor miehei*), SAVINASE® (produced by submerged fermentation of a genetically modified strain of *Bacillus),* e.g., the variants disclosed in the International Patent Application published as WO 92/19729, and DURAZYM® (a protein-engineered variant of SAVINASE®), POLARZYME™, EVERLASE™. All the above-mentioned commercial proteases are available from Novozymes A/S, DK-2880 Bagsværd, Denmark.

Other preferred serine proteases are proteases from *Aspergillus, Bacillus* such as *Bacillus alcalophilus, Bacillus*

*cereus, Bacillus vulgatus, Bacillus mycoide, Rhizopus,* and subtilins from *Bacillus,* especially proteases from the species *Nocardiopsis* such as *Nocardiopsis natto Nocardiopsis dassonvillei* (see, WO 88/03947), especially proteases from the species *Nocardiopsis sp.* NRRL 18262, and *Nocardiopsis dassonvillei* NRRL 18133. Yet other preferred proteases are the serine proteases from mutants of *Bacillus* subtilisins disclosed in the International Patent Application No. PCT/DK89/00002 and WO 91/00345, and the proteases disclosed in EP 415 296.

Another preferred class of proteases is the metalloproteases of microbial origin. Conventional fermented commercial metalloproteases may be used in the methods of the present invention such as is NEUTRASE® (Zn) (produced by submerged fermentation of a strain of *Bacillus subtilis*), available from Novozymes A/S, DK-2880 Bagsværd, Denmark; BACTOSOL® WO and BACTOSOL® SI, available from Sandoz AG, Basle, Switzerland; TOYOZYME®, available from Toyo Boseki Co. Ltd., Japan; and PROTEINASE K® (produced by submerged fermentation of a strain of *Bacillus sp.* KSM-K16), available from Kao Corporation Ltd., Japan.

The protease may be used in a dosage of between 0.005 to 500 mg enzyme protein per L biofilm control solution, preferably between 0.01 to 100 mg enzyme protein per L biofilm control solution.

Lipases

In another preferred embodiment, the other enzyme is a lipase, especially a microbial lipase. As such, the lipase may be selected from yeast, e.g., *Candida;* bacteria, e.g., *Pseudomonas* or *Bacillus;* or filamentous fungi, e.g., *Humicola* or *Rhizomucor.* More specifically, suitable lipases may be the *Rhizomucor miehei* lipase (e.g., prepared as described in EP 238 023), *Thermomyces lanuginosa* lipase e.g., prepared as described in EP 305 216, *Humicola insolens* lipase, *Pseudomonas stutzeri* lipase, *Pseudomonas cepacia* lipase, *Candida antarctica* lipase A or B, or lipases from *rGPL, Absidia blakesleena, Absidia corymbifera, Fusarium solani, Fusarium oxysporum, Penicillum cyclopium, Penicillum crustosum, Penicillum expansum, Rhodotorula glutinis, Thiarosporella phaseolina, Rhizopus microsporus, Sporobolomyces shibatanus, Aureobasidium pullulans, Hansenula anomala, Geotricum penicillatum, Lactobacillus curvatus, Brochothrix thermosohata, Coprinus cinerius, Trichoderma harzanium, Trichoderma reesei, Rhizopus japonicus,* or *Pseudomonas plantari.* Other examples of suitable lipases may be variants of any one of,the lipases mentioned above, e.g., as described in WO 92/05249 or WO 93/11254.

Examples of commercially available lipases include: LIPOLASE™, LIPOLASE ULTRA™, LIPOPRIME™, LIPEX™ from Novozymes, Denmark).

The lipase may be used in a dosage of between 0.005 to 500 mg enzyme protein per L biofilm control solution, preferably between 0.01 to 100 mg enzyme protein per L biofilm control solution.

Cellulases

In another preferred embodiment, the other enzyme is a cellulase or cellulolytic enzyme, which refers to an enzyme which catalyses the degradation of cellulose to glucose, cellobiose, triose and other cellooligosaccharides. Preferably, the cellulase is an endoglucanase, more preferably a microbial endoglucanase, especially a bacterial or fungal endoglucanase. Examples of bacterial endoglucanases are endoglucanases obtained from or producible by bacteria from the group of genera consisting of *Pseudomonas* or *Bacillus lautus.*

The cellulase or endoglucanase may be an acid, neutral, or alkaline cellulase or endoglucanase, i.e., exhibiting maximum cellulolytic activity in the acid, neutral or alkaline pH range, respectively. Accordingly, a useful cellulase or endoglucanase is an acid cellulase or endoglucanase, preferably a fungal acid cellulase or endoglucanase, more preferably a fungal acid cellulase or endoglucanse enzyme with substantial cellulolytic activity under acidic conditions, which is obtained from or producible by fungi from the group consisting of *Trichoderma, Actinomyces, Myrothecium, Aspergillus,* and *Botrytis.*

A preferred acid cellulase or endoglucanase is obtained from the group consisting of *Aspergillus niger, Aspergillus oryzae, Botrytis cinerea, Myrothecium verrucaria, Trichoderma longibrachiatum, Trichoderma reesei,* and *Trichoderma viride.*

Another useful cellulase or endoglucanase is a neutral or alkaline cellulase or endoglucanse, preferably a fungal neutral or alkaline cellulase or endoglucanse, more preferably a fungal alkaline cellulase or endoglucanase with substantial cellulolytic activity under alkaline conditions, which is obtained from fungi selected from the group consisting of *Acremonium, Aspergillus, Chaetomium, Cephalosporium, Fusarium, Gliocladium, Humicola, Irpex, Myceliophthora, Mycogone, Myrothecium, Papulospora, Penicillium, Scopulariopsis, Stachybotrys,* and *Verticillium.*

A preferred alkaline cellulase or endoglucanase is obtained from the group consisting of *Cephalosporium sp., Fusarium oxysporum, Humicola insolens,* or *Myceliopthora thermophila,* or preferably from the group consisting of *Cephalosporium* sp., RYM-202, *Fusarium oxysporum,* DSM 2672, *Humicola insolens,* DSM 1800, or *Myceliopthora thermophila,* CBS 117.65.

In another preferred embodiment, the other enzyme is a xylanase such as an endo-1,3-beta-xylosidase (EC 3.2.1.32), xylan 1,4-beta-xylosidase (EC 3.2.1.37), and alpha-L-arabinofuranosidase (EC 3.2.1.55). Preferably the xylanase is obtained from *Aspergillus aculeatus* (an enzyme exhibiting xylanase activity, which enzyme is immunologically reactive with an antibody raised against a purified xylanase derived from *Aspergillus aculeatus* CBS 101.43, see, for example, WO 94/21785); *Aspergillus oryzae* (see, for example, SU 4610007); *Aureobasidium pullulans* (see, for example, EP 0 373 107 A2); *Bacillus circulans* (WO 91/18978); *Bacillus pumilus* (see, for example, WO 92/03540); *Bacillus stearothermophilus* (see, for example; WO 91/18976, WO 91/10724); *Bacillus sp. AC13* (especially the strain NCIMB 40482, see, for example, WO 94/01532); *Humicola insolens* (see, for example, WO 92/17573); *Rhodothermus* (see, for example, WO 93/08275); *Streptomyces lividans* (see, for example, WO 93/03155); *Streptomyces viridosporus* (see, for example, EP 496 671 A*); Bacillus licheniformis* (see, for example, JP 9213868); *Thermoascus aurantiacus* (see, for example, US patent 4,966,850); *Trichoderma longibrachiatum* and *Chainia sp.* (see, for example, EP 0 353 342 A1); *Trichoderma harzianum* and *Trichoderma reseei* (see, for example, US patent 4,725,544); *Thermomyces lanuginosus* (see, for example, EP 0 456 033 A2); *Thermomonospora fusca* (see, for example, EP 0 473 545 A2); *Trichoderma longibrachiatum* (see W.J.J. van den Tweel et al., Eds., Stability of Enzymes, Proceedings of an International Symposium held in Maastrich, The Netherlands, 22-25 November 1992, Fisk, R.S. and Simpson, pp.323-328); *Dictyoglomus* (see, for example, WO 92/18612); *Streptomyces* (see, for example, US patent 5,116,746); and/or *Thermotoga* (see, for example, WO 93/19171). Other examples of suitable xylanases may be variants (derivatives or homologues) of any one of the above-noted enzymes having xylanolytic activity.

Examples of commercially available cellulase containing products include: NOVOZYM™ 342, CELLUZYME™, CAREZYME™, RENOZYME™ (all Novozymes, Denmark).

The cellulase may be used in a dosage of between 0.005 to 500 mg enzyme protein per L biofilm control solution, preferably between 0.01 to 100 mg enzyme protein per L biofilm control solution.

Pectinases

In another preferred embodiment, the other enzyme is a pectinase such as a polygalacturonase (EC 3.2.1.15), pectinesterase (EC 3.2.1.11), or pectin lyase (EC4.2.2.10). A suitable source organism for pectinases may be *Aspergillus niger.*

In another preferred embodiment, the other enzyme in the alpha-amylase composition comprises a hydrolytic enzyme composition produced by a strain of the fungus *Aspergillus aculeatus,* preferably *Aspergillus aculeatus,* CBS 101.43. It is known that this strain produces an enzyme composition comprising pectinolytic and a range of hemicellulolytic enzyme activities.

Examples of commercially available pectinase containing products include: BioPrep™, SCOURZYME™ and PECTAWASH™ (Novozymes, Denmark).

The pectinase may be used in a dosage of between 0.005 to 500 mg enzyme protein per L biofilm control solution, preferably between 0.01 to 100 mg enzyme protein per L biofilm control solution.

Oxidoreductase

In another embodiment of the invention the alpha-amylase is combined with an oxidoreductase, such as an oxidase, peroxidase, or laccase.

a) Laccases act on molecular oxygen and yield water ($H_2O$) without any need for peroxide (e.g. $H_2O_2$),
b) Oxidases act on molecular oxygen ($O_2$) and yield peroxide ($H_2O_2$), and
c) Peroxidases act on peroxide (e.g. $H_2O_2$) and yield water ($H_2O$).

Examples of laccases (E.C. 1.10.3.2) include laccases derived from a strain of *Polyporus* sp., in particular a strain of *Polyporus pinsitus* or *Polyporus versicolor,* or a strain of *Myceliophthora* sp., in particular *M. thermophila,* a strain of *Scytalidium sp.,* in particular S. *thermophilium,* a strain of *Rhizoctonia* sp., in particular *Rhizoctonia praticola* or *Rhizoctonia solani,* or a strain of a *Rhus* sp., in particular *Rhus vernicifera.* The laccase may also be derived from a fungus such as *Collybia, Fomes, Lentinus, Pleurotus, Aspergillus, Neurospora, Podospora, Phlebia,* e.g. P. *radiata* (WO 92/01046), *Coriolus* sp., e.g., C. *hirsitus* (JP 2-238885), or *Botrytis.*

In specifically contemplated embodiments, the laccase may be selected from the group consisting of: the *Polyporus pinisitus* laccase (also called *Trametes villosa* laccase) described in WO 96/00290, the *Myceliophthora thermophila* laccase described in WO 95/33836, the *Scytalidium thermophilium* laccase described in WO 95/33837, the *Pyricularia oryzae* laccase which can be purchased from SIGMA under the trade name SIGMA no. L5510, the *Coprinus cinereus* laccase described in WO 96/06930, and the *Rhizoctonia solani* laccase described WO 95/07988.

Examples of peroxidases (1.11.1.7) include peroxidases derived from plants (e.g., horseradish peroxidase) or micro-organisms including fungi and bacteria, such as a strain of *Coprinus* sp., such as *Coprinus cinereus* or *Coprinus*

*macrorhizus,* or bacteria such as *Bacillus,* such as *Bacillus pumilus.*

In specifically contemplated embodiments, the peroxidase may be selected from the group consisting of: the *Coprinus cinereus* IFO8371 peroxidase or variants thereof described in WO 95/10602, and the haloperoxidase originating from a strain of *Curvularia verruculosa* CBS 147.63 described in WO 97/04102.

Contemplated oxidases include especially carbohydrate oxidases, which are enzymes classified under EC 1.1.3. Carbohydrate oxidases include glucose oxidase (E.C. 1.1.3.4), hexose oxidase (E.C. 1.1.3.5) xylitol oxidase, galactose oxidase (E.C. 1.1.3.9), pyranose oxidase (E.C. 1.1.3.10), alcohol oxidase (E.C. 1.1.3.13).

Carbohydrate oxidases may be derived from any origin, including, bacterial, fungal, yeast or mammalian origin.

Examples of glucose oxidases include glucose oxidases derived from *Aspergillus* sp., such as a strain of *Aspergillus niger,* or from a strain of *Cladosporium* sp. in particular *Cladosporium oxysporum,* especially *Cl. oxysporum* CBS 163 described in WO 95/29996.

Examples of hexose oxidases include hexose oxidases produced by the red sea-weed *Chondrus crispus* (commonly known as Irish moss) (Sullivan and Ikawa, (1973), Biochim. Biophys. Acts, 309, p. 11-22; Ikawa, (1982), Meth. in Enzymol. 89, carbohydrate metabolism part D, 145-149) that oxidizes a broad spectrum of carbohydrates and the red sea-weed *Iridophycus flaccidum* that produces easily extractable hexose oxidases, which oxidize several different mono- and disaccharides (Bean and Hassid, (1956), J. Biol. Chem, 218, p. 425; Rand et al. (1972, J. of Food Science 37, p. 698-710).

The oxidoreductase may be used in a dosage of between 0.005 to 500 mg enzyme protein per L biofilm control solution, preferably between 0.01 to 100 mg enzyme protein per L biofilm control solution.

In a final aspect the invention relates to the use of a Bacillus alpha-amylase for preventing, removing, reducing, or disrupting biofilm formation on a surface. In a preferred embodiment the alpha-amylase is a *Bacillus* alpha-amylase, preferably one mentioned above in the "Alpha-Amylase" section.

The present invention is further described by the following examples which should not be construed as limiting the scope of the invention.

## Materials & Methods

Chemicals used as buffers and reagents were commercial products of at least reagent grade.

Enzymes:

Alpha-amylase A is a variant alpha-amylase of the parent *Bacillus* sp. alpha-amylase disclosed as SEQ ID NO: 2 in WO 00/60060. The amino acid sequence of said alpha-amylase has the following six amino acid deletions/substitutions:

$$D183^{*}+G184^{*}+R118K+N195F+R320K+R458K.$$

The variant is also disclosed in WO 01/66712. The alkaline alpha-amylase was produced in batch 03AGE014-4.

Alpha-amylase B is derived from a strain of *Bacillus flavothermus* and is disclosed in SEQ ID NO: 4.

Alpha-amylase C is derived from a strain of *Bacillus licheniformis* and is shown as SEQ ID NO: 6 in WO 99/19467.

Protease E is a *Bacillus clausii* (old name: *Bacillus lentus* C360 = NCIB 10309) subtilisin having a M222S substitution covered by EP patent no. 396,608-B1 (Available on request from Novozymes, Denmark).

Lipase A is a lipase variant derived from *Humicola lanuginosa* strain DSM 4109 having the following mutations: T231R, N233R disclosed in US patent no: 6,939,702-B (Available on request from Novozymes).

Cellulase A is a multi-component cellulase from *Humicola insolens* (Available on request from Novozymes, Denmark).

## Bacterial strains.

*Bacillus subtilis* obtained from ATCC 10774.
E. coli ATCC #11229 and ATCC #25922

**Biofilm medium.** Tryptic Soy Broth (TSB, purchased from VWR, P/N DF0370-07) medium was prepared according to the manufacturer's instructions, then diluted to 5% with water. 2 ml of trace elements were added per liter.

**Agar.** Tryptic Soy Agar (TSA, purchased from VWR, P/N DF0369-17) was used as per the manufacturer's directions.

**Trace element solution.** Per liter: 1.5 g $CaCl_2$, 1.0 g $FeSO_4 7.H_2O$, 0.35 g $MnSO_4.2H_2O$, 0.5 g, $NaMoO_4$.

**Stainless steel coupons.** Stainless steel coupons No. 304 were obtained from Metal Samples Company (Munford, AL).

**BioLC Ion Chromatography System.** The IC system consisted of the following components:

GP50 Gradient Pump (P/N 059493)
ED50A Electrochemical Detector (P/N 059499)
AS50 Temperature Controlled Autosampler (P/N 056565)
Electrochemical Cell for Integrated Amperometry, complete with Gold Electrode and Ag/AgCl Reference Electrode (P/N 060386)
Chromelion Data Control Software CHM-1-IC (P/N 060930)

**CDC Biofilm Reactor.** Purchased from Biosurface Technologies, Inc. (P/N CBR 90-2) complete with polycarbonate coupons (24 for each reactor, P/N RD 128-PC).

**Detergent Cleaner Base.** Obtained from Weiman Products (IL, USA) as Burnishine ME - multiple enzyme detergent. The enzymes were denatured prior to use by heating in a microwave on high setting for 1 minute.

Methods:

**Alpha-amylase activity (KNU)**

The amylolytic activity may be determined using potato starch as substrate. This method is based on the break-down of modified potato starch by the enzyme, and the reaction is followed by mixing samples of the starch/enzyme solution with an iodine solution. Initially, a blackish-blue color is formed, but during the break-down of the starch the blue color gets weaker and gradually turns into a reddish-brown, which is compared to a colored glass standard.

One Kilo Novo alpha amylase Unit (KNU) is defined as the amount of enzyme which, under standard conditions (i.e. at 37°C +/- 0.05; 0.0003 M $Ca^{2+}$; and pH 5.6) dextrinizes 5260 mg starch dry substance Merck Amylum solubile.

A folder EB-SM-0009.02/01 describing this analytical method in more detail is available upon request to Novozymes A/S, Denmark, which folder is hereby included by reference.

Determination of degree of identity between two sequences

For purposes of the present invention, the degree of identity between two amino acid sequences is determined by the Clustal method (Higgins, 1989, CABIOS 5: 151-153) using the LASERGENE™ MEGALIGN™ software (DNASTAR, Inc., Madison, WI) with an identity table and the following multiple alignment parameters: Gap penalty of 10, and gap length penalty of 10. Pairwise alignment parameters were Ktuple=1, gap penalty=3, windows=5, and diago-nals=5.

**EXAMPLES**

**Example 1**

Biofilm removal using Alpha-amylase A and Alpha-Amylase C

Biofilm reactors consisted of a 400 ml beaker, a magnetic stirrer, and 2 stainless steel coupons. The coupons are taped vertically to the sides of the beaker so that the bottom edge of the coupon rested on the bottom of the beaker. A stir bar is added and the beakers are covered with a circle of aluminum foil and autoclaved. 200 ml of sterile biofilm medium is added to each beaker. To prepare the inoculum, each bacterial strain (from *Bacillus subtilis)* is grown overnight at 28°C on plate count agar. Using a sterile swab, each is suspended in sterile water to an $OD_{686}$ of 0.100 and then diluted additionally to $10^{-1}$. Each assay consisted of 4 control beakers without enzyme, 2 beakers with 50 mg of enzyme protein per liter of solution, and 2 beakers with 100 mg of enzyme protein per liter of solution. Beakers are first incubated at 37°C overnight with stirring to grow the biofilm on the stainless steel coupons. Following this incubation step, the enzymes are added in the 2 dosages noted above as per the table below and incubated for an additional 2 hours at 40°C. Thereafter, each beaker and stainless steel coupon is rinsed carefully with sterile water, stained with crystal violet, rinsed with steile water, the remaining biofilm solubilized with acetic acid, and the absorbance of an aliquot of each solution is measured at 600nm using a spectrophotometer. The measured absorbances of the solutions provide a direct indication of the amount of biofilm remaining on the stainless steel coupons. A low absorbance corresponds to a good enzyme effect and little remaining biofilm, whereas a high absorbance corresponds to a poor (or lack thereof) enzyme effect and considerable remaining biofilm.

| Sample # | Enzyme Used | Enzyme Protein Conc. |
|---|---|---|
| 1 | Alpha-amylase A | 50 mg per L & 100 mg per L |
| 2 | Alpha-Amylase C | 50 mg per L & 100 mg per L |
| 3 | No enzyme (control) | NA |

**Example 2**

Raw starch solubilization using Alpha-Amylase A, B, and C

The rate at which various alpha-amylases solubilize raw, unhydrated wheat starch was measured. Alpha-Amylase A, B and C, respectively, were used in the study.

Twenty five milliliters of a 1% raw wheat starch solution with pH 8 tris buffer and 15°dH was poured into a tube with lid and placed in a 40°C water bath. The starting level of "reducing ends" was measured prior to addition of enzyme. The enzyme concentration used in the study was 3 mg enzyme protein per g raw wheat starch. One milliliter samples were taken out at different times. Twenty microliters of 1 M HCl was added prior to incubation at 99°C for 10 minutes. The combination of acid and heat inactivates the amylase. Then 20 microL 1 M NaOH was added to make sure the sample was no longer acidic. The sample was then diluted, incubated with color reagent (PHABH, potassium sodium tartrate, NaOH) at 95°C for 10 minutes and finally centrifuged before measuring the OD at 410 nm on the supernatant. The control (100% hydrolyzed starch) was made by incubating a solution of 1% raw wheat starch in 1M HCl in an oven at 110°C for 4 hours. This treatment was used to calculate the maximum amount of glucose which could be produced per gram of the raw wheat starch. This value was set to 100% in the graph shown in Fig. 1.

For Alpha-Amylase A and B it can be seen that the initial rate of raw wheat starch solubilization observed within the first 5 hours is significantly more rapid than for Alpha-Amylase C. This resulted in a greater percentage of the starch being solubilized by the former two alpha-amylases vs. for the latter over this time period.

**Example 3**

Biofilm removal using Alpha-amylase A and C in combination with protease E and detergent

A mono-component biofilm of *Escherichia coli* (ATCC #11229) is grown on polycarbonate coupons in a pre-sterilized CDC biofilm reactor. At the start of the experiment, cultures of *E. coli* were grown on tryptic soy agar (TSA) overnight at 37°C. The next morning, a single colony was picked from the plate using a 1 microL sterile inoculation loop and added to a solution of 40 g TSB/liter of water. This solution was incubated at 37°C overnight to grow up the culture. The following day, 1 milliliter of this culture was added to 400 milliliters of minimal media (0.30 g TSB/liter sterile water) contained in the CDC biofilm reactor. The solution was slowly stirred at 130 rpm and grown for 2 days at 22°C in a non fed batch mode. After the 2 day growth period, the coupon holder rods and coupons were removed from the reactor, rinsed in sterile dilution water to remove planktonic cells, the coupons were carefully removed from the rods and then incubated at 40°C for 1 hour in the following solutions (30 milliliters each).

A: Detergent cleaner base alone, 0.21g detergent in sterile water
B: Detergent cleaner base, 0.21 g + 0.51 mg enzyme protein Protease E + 0.06 mg enzyme protein Alpha-amylase A
C: Detergent cleaner base, 0.21g + 0.51 mg enzyme protein Protease E + 0.16 mg enzyme protein Alpha-amylase C.

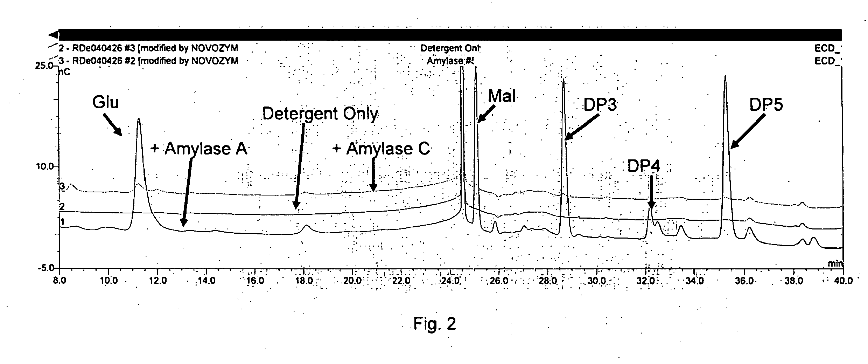

Following the incubation step, the coupons were removed. The solutions were filtered through 0.45□micro m Nylon syringe filters and their sugar contents measured by Ion Chromatography. PA100 guard and analytical columns (P/N 043055) were used for the separation. A mobile phase gradient between 60/40 deionized water/100mM NaOH and 100% 100mM NaOH/1M Sodium Acetate (exponential gradient started 10 minutes into the separation and ended at 85 minutes) was used to effect the separation. Fig. 2 shows an overlay of the 3 chromatograms generated in this experiment. When Alpha-Amylase A is used, a significantly higher level of low molecular weight sugars (glucose = glu, maltose = mal, maltotriose = DP3, maltotetraose = DP4, maltopentaose = DP5) were generated versus with the detergent alone or with Alpha-Amylase C. This indicated an increased level of biofilm removal through increased breakdown of the amylopectin exopolysaccharides produced by the *Ecoli* bacteria.

**Example 4**

Biofilm removal using Alpha-amylase A and C in combination with protease E, Cellulase A, Lipase A and detergent.

Two CDC biofilm reactors fitted with polycarbonate coupons were autoclaved and filled with sterile 1/10 strength tryptic soy broth (TSB, 3g/liter strength) and inoculated with 1 ml log phase culture of *Escherichia coli* (ATCC # 25922). The initial cell count in the reactors averaged to $5 \times 10^8$ cfu/mL. Both reactors were operated for 24 hours in batch mode at 37°C (no inflow or outflow). After this period, continuous flow of the 1/10 TSB was started at a flowrate of 12 ml/min at 37°C. The E. *coli* biofilm was grown for 4 days. After this time, 1 rod from each reactor (labeled as reactor 1 or 2) was pulled and placed into sterile glass beakers containing two hundred milliliters of the following filter-sterilized solutions.

A: Detergent cleaner base alone, 1.4 g detergent in sterile water
B: Detergent cleaner base, 1.4 g + 3.4 mg enzyme protein Protease E + 0.48 mg enzyme protein Lipase A + 0.23 mg enzyme protein Cellulase A + 0.40 mg enzyme protein Alpha-amylase A
C: Detergent cleaner base, 1.4 g + 3.4 mg enzyme protein Protease E+ 0.48 mg enzyme protein Lipase A + 0.23 mg enzyme protein Cellulase A + 0.40 mg enzyme protein Alpha-amylase C.

The solutions in each of the beakers were incubated at 40°C with moderate stirring for 30 minutes, after which each of the rods were gently rinsed in sterile water. Finally, *E. coli* was enumerated on 2 of the 3 coupons from each rod using tryptic soy agar (TSA). The average plate count results obtained from the study were as follows:

| Treatment | Average $\log_{10}$ cfu/cm$^2$ on coupons |
|---|---|
| A | $4 \times 10^7$ |
| B | $3 \times 10^4$ |
| C | $2 \times 10^5$ |

## Claims

1. A method for preventing, removing, reducing or disrupting biofilm present on a surface, comprising contacting the surface with an alpha-amylase derived from a bacterium, where said alpha- amylase has alpha-amylase activity and an amino acid sequence having at least 80% identity with SEQ ID NO:2, and wherein the alpha-amylase is selected from the group consisting of:

   a) an alpha-amylase comprising an amino acid sequence having a deletion in positions D183 and/or G184 using SEQ ID NO:2 for numbering;
   b) an alpha-amylase comprising an amino acid sequence having deletions in positions D183+G184 using SEQ ID NO:2 for numbering; and
   c) an alpha-amylase comprising an amino acid sequence having one or more of substitutions selected from the group consisting of: R118K, N195F, R320K, and R458K, and having a deletion in positions D183 and/or G184 using SEQ ID NO:2 for numbering.

2. The method of claim 1, wherein the biofilm contaminated or prone surface is contacted for between 1 minute and 2 days.

3. The method of any of claims 1-2, wherein the alpha-amylase comprises Asn-Gly-Thr-Met-Met-Gln-Tyr-Phe-Glu-Trp in its N-terminal amino acid region.

4. The method of any of claims 1-3, wherein the alpha-amylase is used in a concentration of between 0.005-500 mg enzyme protein per L biofilm control solution.

5. The method of any of claims 1-4, wherein the alpha-amylase has a percentage (%) of hydrolyzed starch that is higher than 15, after 5 hours at 40°C, 3 mg enzyme protein per g starch, pH 8.0.

6. The method of any of claims 1-5, wherein further enzymes are present, which enzymes are selected from the group consisting of an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidoreductases, pectinolytic enzyme, peptidoglutaminase, peroxidase, phytase, polyphenoloxidase, proteolytic enzyme, ribonuclease, transglutaminase, or xylanase.

7. The method of any of claims 1-6, wherein further one or more agents selected from the group consisting of dispersants, surfactants, anti-microbials, and biocides, are present.

8. The method of any of claims 1-7, wherein the surface is a hard, soft, or porous surface.

9. The method of claim 8, wherein the surface is a membrane.

10. The method of any of claims 1-9, wherein the biofilm removal is done at a temperature between 10-70°C.

**11.** Use of the alpha-amylase of claim 1 for prevention or removal of biofilm from surfaces.

**12.** The use of claim 11 wherein further enzymes and/or agents as defined in claims 1-10 are used.

**13.** The method of claim 1, comprising contacting the surface with an alpha-amylase derived from a bacterium, said alpha amylase has alpha-amylase activity and an amino acid sequence having at least 90% identity with SEQ ID NO:2, and wherein the alpha-amylase is selected from the group consisting of:

a) an alpha-amylase comprising an amino acid sequence having a deletion in positions D183 and/or G184 using SEQ ID NO:2 for numbering;
b) an alpha-amylase comprising an amino acid sequence having deletions in positions D183+G184 using SEQ ID NO:2 for numbering; and
c) an alpha-amylase comprising an amino acid sequence having one or more of substitutions selected from the group consisting of: R118K, N195F, R320K, and R458K, and having a deletion in positions D183 and/or G184 using SEQ ID NO:2 for numbering.

**14.** The method in accordance with claim 13 wherein the alpha-amylase comprises an amino acid sequence having at least 95% identity with SEQ ID NO:2 and a deletion in positions D183 and/or G184 using SEQ ID NO:2 for numbering.

**Patentansprüche**

**1.** Verfahren zum Vorbeugen, Entfernen, Reduzieren oder Stören von auf einer Oberfläche vorhandenem Biofilm, umfassend das In-Kontakt-Bringen der Oberfläche mit einer aus einem Bakterium abgeleiteten alpha-Amylase, wobei die alpha-Amylase alpha-Amylaseaktivität und eine Aminosäuresequenz mit mindestens 80% Identität zu SEQ ID NO: 2 aufweist, und wobei die alpha-Amylase ausgewählt ist aus der Gruppe bestehend aus:

a) einer alpha-Amylase, umfassend eine Aminosäuresequenz mit einer Deletion in Positionen D183 und/oder G184 unter Verwendung von SEQ ID NO: 2 zur Nummerierung;
b) einer alpha-Amylase, umfassend eine Aminosäuresequenz mit Deletionen in Positionen D183+G184 unter Verwendung von SEQ ID NO: 2 zur Nummerierung; und
c) einer alpha-Amylase, umfassend eine Aminosäuresequenz mit einer oder mehreren Substitutionen, ausgewählt aus der Gruppe bestehend aus: R118K, N195F, R320K und R458K, und mit einer Deletion in Positionen D183 und/oder G184 unter Verwendung von SEQ ID NO: 2 zur Nummerierung.

**2.** Verfahren nach Anspruch 1, wobei die Biofilm kontaminierte oder gefährdete Oberfläche für zwischen 1 Minute und 2 Tage in Kontakt gebracht wird.

**3.** Verfahren nach einem beliebigen der Ansprüche 1-2, wobei die alpha-Amylase Asn-Gly-Thr-Met-Met-Gln-Tyr-Phe-Glu-Trp in dessen N-terminaler Aminosäureregion umfasst.

**4.** Verfahren nach einem beliebigen der Ansprüche 1-3, wobei die alpha-Amylase in einer Konzentration von zwischen 0,005-500 mg Enzymprotein pro L Biofilm-Kontrolllösung verwendet wird.

**5.** Verfahren nach einem beliebigen der Ansprüche 1-4, wobei die alpha-Amylase eine prozentuale (%) hydrolysierte Stärke aufweist, die nach 5 Stunden bei 40°C, 3 mg Enzymprotein pro g Stärke, pH 8,0, höher als 15 ist.

**6.** Verfahren nach einem beliebigen der Ansprüche 1-5, wobei weitere Enzyme vorhanden sind, welche Enzyme ausgewählt sind aus der Gruppe bestehend aus einer Aminopeptidase, einer Amylase, einer Carbohydrase, einer Carboxypeptidase, einer Catalase, einer Cellulase, einer Chitinase, einer Cutinase, einer Cyclodextringlycosyltransferase, einer Desoxyribonuklease, einer Esterase, einer alpha-Galactosidase, einer beta-Galactosidase, einer Glucoamylase, einer alpha-Glucosidase, einer beta-Glucosidase, einer Haloperoxidase, einer Invertase, einer Laccase, einer Lipase, einer Mannosidase, Oxidoreduktasen, einem pektinolytischen Enzym, einer Peptidglutaminase, einer Peroxidase, einer Phytase, einer Polyphenoloxidase, einem proteolytischen Enzym, einer Ribonuklease, einer Transglutaminase oder einer Xylanase.

**7.** Verfahren nach einem beliebigen der Ansprüche 1-6, wobei des Weiteren ein oder mehrere Mittel vorhanden sind, ausgewählt aus der Gruppe bestehend aus Dispersionsmitteln, oberflächenaktiven Mitteln, anti-mikrobielle Mittel und Bioziden.

**8.** Verfahren nach einem beliebigen der Ansprüche 1-7, wobei die Oberfläche eine harte, weiche oder poröse Oberfläche ist.

**9.** Verfahren nach Anspruch 8, wobei die Oberfläche eine Membran ist.

**10.** Verfahren nach einem beliebigen der Ansprüche 1-9, wobei die Biofilmentfernung bei einer Temperatur zwischen 10-70 °C durchgeführt wird.

**11.** Verwendung der alpha-Amylase gemäß Anspruch 1 zur Vorbeugung oder Entfernung von Biofilm von Oberflächen.

**12.** Verwendung nach Anspruch 11, wobei weitere wie in Ansprüchen 1-10 definierte Enzyme und/oder Mittel verwendet werden.

**13.** Verfahren nach Anspruch 1, umfassend das In-Kontakt-Bringen der Oberfläche mit einer aus einem Bakterium abgeleiteten alpha-Amylase, wobei die alpha-Amylase alpha-Amylaseaktivität und eine Aminosäuresequenz mit mindestens 90% Identität zu SEQ ID NO: 2 aufweist, und wobei die alpha-Amylase ausgewählt ist aus der Gruppe bestehend aus:

a) einer alpha-Amylase, umfassend eine Aminosäuresequenz mit einer Deletion in Positionen D183 und/oder G184 unter Verwendung von SEQ ID NO: 2 zur Nummerierung;
b) einer alpha-Amylase, umfassend eine Aminosäuresequenz mit Deletionen in Positionen D183+G184 unter Verwendung von SEQ ID NO: 2 zur Nummerierung; und
c) einer alpha-Amylase, umfassend eine Aminosäuresequenz mit einer oder mehreren Substitutionen, ausgewählt aus der Gruppe bestehend aus: R118K, N195F, R320K und R458K, und mit einer Deletion in Positionen D183 und/oder G184 unter Verwendung von SEQ ID NO: 2 zur Nummerierung.

**14.** Verfahren nach Anspruch 13, wobei die alpha-Amylase eine Aminosäuresequenz mit mindestens 95% Identität zu SEQ ID NO: 2 und eine Deletion in Positionen D183 und/oder G184 unter Verwendung von SEQ ID NO: 2 zur Nummerierung umfasst.

## Revendications

**1.** Méthode de prévention, d'élimination, de réduction, ou de désintégration d'un biofilm présent sur une surface, comprenant la mise en contact de la surface avec une alpha-amylase dérivée d'une bactérie, où ladite alpha-amylase possède une activité alpha-amylase et a une séquence d'acides aminés présentant au moins 80 % d'identité de séquence avec SEQ ID NO : 2, et où l'alpha-amylase est choisie dans le groupe constitué de :

a) une alpha-amylase comprenant une séquence d'acides aminés comportant une délétion dans les positions D183 et/ou G184 en utilisant SEQ ID NO : 2 pour la numérotation ;
b) une alpha-amylase comprenant une séquence d'acides aminés comportant une délétion dans les positions D183+G184 en utilisant SEQ ID NO : 2 pour la numérotation ; et
c) une alpha-amylase comprenant une séquence d'acides aminés comportant une ou plusieurs des substitutions choisies dans le groupe constitué de : R118K, N195F, R320K, et R485K, et comportant une délétion dans les positions D183 et/ou G184 en utilisant SEQ ID NO : 2 pour la numérotation.

**2.** Méthode selon la revendication 1, dans laquelle la surface contaminée par un biofilm ou encline à être contaminée par un biofilm est mise en contact pendant entre 1 minute et 2 jours.

**3.** Méthode selon l'une quelconque des revendications 1 ou 2, dans laquelle l'alpha-amylase comprend Asn-Gly-Thr-Met-Met-Gln-Tyr-Phe-Glu-Trp dans sa région des acides aminés N-terminaux.

**4.** Méthode selon l'une quelconque des revendications 1 à 3, dans laquelle l'alpha-amylase est utilisée dans une concentration entre 0,005 et 500 mg de protéine enzyme par 1 de solution contrôle de biofilm.

**5.** Méthode selon l'une quelconque des revendications 1 à 4, dans laquelle l'alpha-amylase a un pourcentage (%) d'amidon hydrolysé qui est supérieur à 15, après 5 heures à 40 °C, 3 mg de protéine enzyme par g d'amidon, pH 8,0.

6. Méthode selon l'une quelconque des revendications 1 à 5, dans laquelle d'autres enzymes sont présentes, lesquelles enzymes sont choisies dans le groupe constitué d'une aminopeptidase, amylase, carbohydrase, carboxypeptidase, catalase, cellulase, chitinase, cutinase, cyclodextrine glycosyltransférase, désoxyribonucléase, estérase, alpha-galactosidase, bêta-galactosidase, glucoamylase, alpha-glucosidase, bêta-glucosidase, halogénoperoxydase, invertase, laccase, lipase, mannosidase, oxydoréductases, enzyme pectinolytique, peptidoglutaminase, peroxydase, phytase, polyphénoloxydase, enzyme protéolytique, ribonucléase, transglutaminase, ou xylanase.

7. Méthode selon l'une quelconque des revendications 1 à 6, dans laquelle en outre un ou plusieurs agents choisis dans le groupe constitué d'agents dispersants, surfactants, antimicrobiens et biocides, sont présents.

8. Méthode selon l'une quelconque des revendications 1 à 7, dans laquelle la surface est une surface dure, molle ou poreuse.

9. Méthode selon la revendication 8, dans laquelle la surface est une membrane.

10. Méthode selon l'une quelconque des revendications 1 à 9, dans laquelle l'élimination du biofilm est réalisée à une température entre 10 et 70 °C.

11. Utilisation de l'alpha-amylase selon la revendication 1, pour la prévention ou l'élimination d'un biofilm à partir de surfaces.

12. Utilisation selon la revendication 11, où d'autres enzymes et/ou agents tels que définis dans les revendications 1 à 10 sont utilisés.

13. Méthode selon la revendication 1, comprenant la mise en contact de la surface avec une alpha-amylase dérivée d'une bactérie, ladite alpha-amylase possède une activité alpha-amylase et a une séquence d'acides aminés présentant au moins 90 % d'identité avec SEQ ID NO : 2, et où l'alpha-amylase est choisie dans le groupe constitué de :

a) une alpha-amylase comprenant une séquence d'acides aminés comportant une délétion dans les positions D183 et/ou G184 en utilisant SEQ ID NO : 2 pour la numérotation ;
b) une alpha-amylase comprenant une séquence d'acides aminés comportant une délétion dans les positions D183+G184 en utilisant SEQ ID NO : 2 pour la numérotation ; et
c) une alpha-amylase comprenant une séquence d'acides aminés comportant une ou plusieurs des substitutions choisies dans le groupe constitué de : R118K, N195F, R320K, et R485K, et comportant une délétion dans les positions D183 et/ou G184 en utilisant SEQ ID NO : 2 pour la numérotation.

14. Méthode selon la revendication 13, dans laquelle l'alpha-amylase comprend une séquence d'acides aminés présentant au moins 95 % d'identité de séquence avec SEQ ID NO : 2 et une délétion dans les positions D183 et/ou G184 en utilisant SEQ ID NO : 2 pour la numérotation.

Fig. 1

Fig. 2

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0022103 A
- WO 9920239 A
- WO 9826807 A
- WO 0198214 A
- WO 0166712 A
- WO 0060060 A
- US 10877847 B
- WO 9526397 A
- WO 9700324 A
- EP 1022334 A
- WO 9219729 A
- WO 8803947 A
- DK 8900002 W
- WO 9100345 A
- EP 415296 A
- EP 238023 A
- EP 305216 A
- WO 9205249 A
- WO 9311254 A
- WO 9421785 A
- EP 0373107 A2
- WO 9118978 A
- WO 9203540 A
- WO 9118976 A
- WO 9110724 A
- WO 9401532 A
- WO 9217573 A
- WO 9308275 A
- WO 9303155 A
- EP 496671 A
- JP 9213868 B
- US 4966850 A
- EP 0353342 A1
- US 4725544 A
- EP 0456033 A2
- EP 0473545 A2
- WO 9218612 A
- US 5116746 A
- WO 9319171 A
- WO 9201046 A
- JP 2238885 A
- WO 9600290 A
- WO 9533836 A
- WO 9533837 A
- WO 9606930 A
- WO 9507988 A
- WO 9510602 A
- WO 9704102 A
- WO 9529996 A
- WO 9919467 A
- EP 396608 B1
- US 6939702 B

**Non-patent literature cited in the description**

- **COSTERTON et al.** *Science,* 1999, vol. 284, 1318-1322
- **TSUKAMOTO et al.** *Biochemical and Biophysical Research Communications,* 1988, vol. 151, 25-31
- **PALMER ; WHITE.** *Trends in Microbiology,* 1997, vol. 5, 435-440
- **COSTERTON et al.** *Annual Reviews of Microbiology,* 1987, vol. 41, 435-464
- **MUELLER.** *TAPPI Proceedings, 1994 Biological Sciences Symposium,* 1994, 195-201
- **MCELDOWNEY ; FLETCHER.** *Journal of General Microbiology,* 1986, vol. 132, 513-523
- **SUTHERLAND.** Surface Carbohydrates of the Prokaryotic Cell. Academic Press, 1977, 27-96
- Stability of Enzymes, Proceedings of an International Symposium held in Maastrich, The Netherlands. 22 November 1992
- **SULLIVAN ; IKAWA.** *Biochim. Biophys. Acts,* 1973, vol. 309, 11-22
- **IKAWA.** *Meth. in Enzymol.,* 1982, vol. 89, 145-149
- **BEAN ; HASSID.** *J. Biol. Chem,* 1956, vol. 218, 425
- **RAND et al.** *J. of Food Science,* 1972, vol. 37, 698-710
- **HIGGINS.** *CABIOS,* 1989, vol. 5, 151-153